# EUROPEAN PATENT APPLICATION

(11) **EP 2 550 982 A1**
(43) Date of publication of application: **30.01.2013**
(21) Application number: 12178076.1
(22) Date of filing: 26.07.2012
(51) Int. Cl.: A61L 27/34, A61L 27/50, A61F 2/00

(54) **Devices for surgical applications**

(30) Priority: 27.07.2011 US 201161512011 P
(71) Applicant: Technion Research & Development Foundation Ltd., 32000 Haifa (IL); Health Corporation - Rambam, 31096 Haifa (IL)
(72) Inventor: Itskovitz-Eldor, Joseph, 34987 Haifa (IL); Zussman, Eyal, 34354 Haifa (IL); Lowenstein, Lior, 3491914 Haifa (IL); Zeevi-Levin, Naama, 2850142 Kiryat-Ata (IL); Eldor, Liron, 34987 Haifa (IL)
(74) Representative: Dennemeyer & Associates S.A.

(57) **Abstract**

Provided is a device comprising at least two layers, said at least two layers being at least partially overlapping (e.g., superposed) and contacting one another, wherein a first layer of said at least two layers comprises a non-biodegradable mesh, and wherein a second layer of said at least two layers comprises an electrospun element, and wherein the device is devoid of an extracellular matrix generated by mesenchymal progenitor cells, which are characterized by a reduced differentiation potential into an adipogenic lineage by at least about 50% as compared to differentiation potential of mesenchymal stem cells from an adult adipose source under identical assay conditions, and by an increased osteogenic differentiation potential by at least about 20% as compared to the osteogenic differentiation potential of adipose-derived MSCs under identical assays conditions.

SEQUENCE LISTING

<110>  Technion Research & Development Foundation Ltd.
       Health Corporation - Rambam
      Itskovitz-Eldor, Joseph
      Zussman, Eyal
      Lowenstein, Lior
      Zeevi-Levin, Naama
      Eldor, Liron

<120>  DEVICES FOR SURGICAL APPLICATIONS

<130>  54480

<150>  US 61/512,011
<151>  2011-07-27

<160>  8

<170>  PatentIn version 3.5

<210>  1
<211>  1818
<212>  DNA
<213>  Homo sapiens

<400>  1
ggcgcccgcg cccgcccccg cgccgggccc ggctcggccc gacccggctc cgccgcgggc      60

aggcggggcc cagcgcactc ggagcccgag cccgagccgc agccgccgcc tggggcgctt     120

gggtcggcct cgaggacacc ggagaggggc gccacgccgc cgtggccgca gaaatgacca     180

tggttgacac agagatgcca ttctggccca ccaactttgg gatcagctcc gtggatctct     240

ccgtaatgga agaccactcc cactcctttg atatcaagcc cttcactact gttgacttct     300

ccagcatttc tactccacat tacgaagaca ttccattcac aagaacagat ccagtggttg     360

cagattacaa gtatgacctg aaacttcaag agtaccaaag tgcaatcaaa gtggagcctg     420

catctccacc ttattattct gagaagactc agctctacaa taagcctcat gaagagcctt     480

ccaactccct catggcaatt gaatgtcgtg tctgtggaga taaagcttct ggatttcact     540

atggagttca tgcttgtgaa ggatgcaagg gtttcttccg gagaacaatc agattgaagc     600

ttatctatga cagatgtgat cttaactgtc ggatccacaa aaaaagtaga aataaatgtc     660

agtactgtcg gtttcagaaa tgccttgcag tggggatgtc tcataatgcc atcaggtttg     720

ggcggatgcc acaggccgag aaggagaagc tgttggcgga gatctccagt gatatcgacc     780

agctgaatcc agagtccgct gacctccggg ccctggcaaa acatttgtat gactcataca     840

taaagtcctt cccgctgacc aaagcaaagg cgagggcgat cttgacagga aagacaacag     900

acaaatcacc attcgttatc tatgacatga attccttaat gatgggagaa gataaaatca     960

agttcaaaca catcaccccc ctgcaggagc agagcaaaga ggtggccatc cgcatctttc    1020

agggctgcca gtttcgctcc gtggaggctg tgcaggagat cacagagtat gccaaaagca    1080

ttcctggttt tgtaaatctt gacttgaacg accaagtaac tctcctcaaa tatggagtcc    1140

acgagatcat ttacacaatg ctggcctcct tgatgaataa agatggggtt ctcatatccg 1200

agggccaagg cttcatgaca agggagtttc taaagagcct gcgaaagcct tttggtgact 1260

ttatggagcc caagtttgag tttgctgtga agttcaatgc actggaatta gatgacagcg 1320

acttggcaat atttattgct gtcattattc tcagtggaga ccgcccaggt ttgctgaatg 1380

tgaagcccat tgaagacatt caagacaacc tgctacaagc cctggagctc cagctgaagc 1440

tgaaccaccc tgagtcctca cagctgtttg ccaagctgct ccagaaaatg acagacctca 1500

gacagattgt cacggaacac gtgcagctac tgcaggtgat caagaagacg gagacagaca 1560

tgagtcttca cccgctcctg caggagatct acaaggactt gtactagcag agagtcctga 1620

gccactgcca acatttccct tcttccagtt gcactattct gagggaaaat ctgacaccta 1680

agaaatttac tgtgaaaaag cattttaaaa agaaaaggtt ttagaatatg atctatttta 1740

tgcatattgt ttataaagac acatttacaa tttactttta atattaaaaa ttaccatatt 1800

atgaaattgc tgatagta 1818

<210> 2
<211> 1820
<212> DNA
<213> Homo sapiens

<400> 2
ttcaagtctt tttcttttaa cggattgatc ttttgctaga tagagacaaa atatcagtgt 60

gaattacagc aaacccctat tccatgctgt tatgggtgaa actctgggag attctcctat 120

tgacccagaa agcgattcct tcactgatac actgtctgca aacatatcac aagaaatgac 180

catggttgac acagagatgc cattctggcc caccaacttt gggatcagct ccgtggatct 240

ctccgtaatg gaagaccact cccactcctt tgatatcaag cccttcacta ctgttgactt 300

ctccagcatt tctactccac attacgaaga cattccattc acaagaacag atccagtggt 360

tgcagattac aagtatgacc tgaaacttca agagtaccaa agtgcaatca aagtggagcc 420

tgcatctcca ccttattatt ctgagaagac tcagctctac aataagcctc atgaagagcc 480

ttccaactcc ctcatggcaa ttgaatgtcg tgtctgtgga gataaagctt ctggatttca 540

ctatggagtt catgcttgtg aaggatgcaa gggtttcttc cggagaacaa tcagattgaa 600

gcttatctat gacagatgtg atcttaactg tcggatccac aaaaaaagta gaaataaatg 660

tcagtactgt cggtttcaga aatgccttgc agtggggatg tctcataatg ccatcaggtt 720

tgggcggatg ccacaggccg agaaggagaa gctgttggcg gagatctcca gtgatatcga 780

ccagctgaat ccagagtccg ctgacctccg ggccctggca aaacatttgt atgactcata 840

cataaagtcc ttcccgctga ccaaagcaaa ggcgagggcg atcttgacag gaaagacaac 900

agacaaatca ccattcgtta tctatgacat gaattcctta atgatgggag aagataaaat 960

caagttcaaa cacatcaccc ccctgcagga gcagagcaaa gaggtggcca tccgcatctt 1020

tcagggctgc cagtttcgct ccgtggaggc tgtgcaggag atcacagagt atgccaaaag 1080

cattcctggt tttgtaaatc ttgacttgaa cgaccaagta actctcctca aatatggagt 1140

ccacgagatc atttacacaa tgctggcctc cttgatgaat aaagatgggg ttctcatatc 1200

cgagggccaa ggcttcatga caagggagtt tctaaagagc ctgcgaaagc cttttggtga 1260

ctttatggag cccaagtttg agtttgctgt gaagttcaat gcactggaat tagatgacag 1320

cgacttggca atatttattg ctgtcattat tctcagtgga gaccgcccag gtttgctgaa 1380

tgtgaagccc attgaagaca ttcaagacaa cctgctacaa gccctggagc tccagctgaa 1440

gctgaaccac cctgagtcct cacagctgtt tgccaagctg ctccagaaaa tgacagacct 1500

cagacagatt gtcacggaac acgtgcagct actgcaggtg atcaagaaga cggagacaga 1560

catgagtctt cacccgctcc tgcaggagat ctacaaggac ttgtactagc agagagtcct 1620

gagccactgc caacatttcc cttcttccag ttgcactatt ctgagggaaa atctgacacc 1680

taagaaattt actgtgaaaa agcattttaa aaagaaaagg ttttagaata tgatctattt 1740

tatgcatatt gtttataaag acacatttac aatttacttt taatattaaa aattaccata 1800

ttatgaaatt gctgatagta 1820

<210> 3
<211> 1919
<212> DNA
<213> Homo sapiens

<400> 3
ttggtggaag gtgggtgtgt agtcgtggta ctttacgcct cggtgtttag ggaggagcct 60

aaggtaagga gtcagaaacg gggagtaacc gagctgcggc ttttatataa ggtcagtggt 120

aggtaaggaa ggggccttaa cctctgctgg tgaccagaag cctgcatttc tgcattctgc 180

ttaattccct ttccttagat ttgaaagaag ccaacactaa accacaaata tacaacaagg 240

ccattttctc aaacgagagt cagcctttaa cgaaatgacc atggttgaca cagagatgcc 300

attctggccc accaactttg ggatcagctc cgtggatctc tccgtaatgg aagaccactc 360

ccactccttt gatatcaagc ccttcactac tgttgacttc tccagcattt ctactccaca 420

ttacgaagac attccattca caagaacaga tccagtggtt gcagattaca agtatgacct 480

gaaacttcaa gagtaccaaa gtgcaatcaa agtggagcct gcatctccac cttattattc 540

tgagaagact cagctctaca ataagcctca tgaagagcct tccaactccc tcatggcaat 600

tgaatgtcgt gtctgtggag ataaagcttc tggatttcac tatggagttc atgcttgtga 660

aggatgcaag ggtttcttcc ggagaacaat cagattgaag cttatctatg acagatgtga 720

tcttaactgt cggatccaca aaaaaagtag aaataaatgt cagtactgtc ggtttcagaa 780

atgccttgca gtggggatgt ctcataatgc catcaggttt gggcggatgc cacaggccga 840

gaaggagaag ctgttggcgg agatctccag tgatatcgac cagctgaatc cagagtccgc 900

tgacctccgg gccctggcaa aacatttgta tgactcatac ataaagtcct tcccgctgac 960

caaagcaaag gcgagggcga tcttgacagg aaagacaaca gacaaatcac cattcgttat 1020

ctatgacatg aattccttaa tgatgggaga agataaaatc aagttcaaac acatcacccc 1080

cctgcaggag cagagcaaag aggtggccat ccgcatcttt cagggctgcc agtttcgctc 1140

cgtggaggct gtgcaggaga tcacagagta tgccaaaagc attcctggtt ttgtaaatct 1200

tgacttgaac gaccaagtaa ctctcctcaa atatggagtc cacgagatca tttacacaat 1260

gctggcctcc ttgatgaata aagatggggt tctcatatcc gagggccaag gcttcatgac 1320

aagggagttt ctaaagagcc tgcgaaagcc ttttggtgac tttatggagc ccaagtttga 1380

gtttgctgtg aagttcaatg cactggaatt agatgacagc gacttggcaa tatttattgc 1440

tgtcattatt ctcagtggag accgcccagg tttgctgaat gtgaagccca ttgaagacat 1500

tcaagacaac ctgctacaag ccctggagct ccagctgaag ctgaaccacc ctgagtcctc 1560

acagctgttt gccaagctgc tccagaaaat gacagacctc agacagattg tcacggaaca 1620

cgtgcagcta ctgcaggtga tcaagaagac ggagacagac atgagtcttc acccgctcct 1680

gcaggagatc tacaaggact tgtactagca gagagtcctg agccactgcc aacatttccc 1740

ttcttccagt tgcactattc tgagggaaaa tctgacacct aagaaattta ctgtgaaaaa 1800

gcattttaaa aagaaaaggt tttagaatat gatctatttt atgcatattg tttataaaga 1860

cacatttaca atttactttt aatattaaaa attaccatat tatgaaattg ctgatagta 1919

<210> 4
<211> 1892
<212> DNA
<213> Homo sapiens

<400> 4
ggcgcccgcg cccgcccccg cgccgggccc ggctcggccc gacccggctc cgccgcgggc 60

aggcggggcc cagcgcactc ggagcccgag cccgagccgc agccgccgcc tggggcgctt 120

gggtcggcct cgaggacacc ggagaggggc gccacgccgc cgtggccgca gatttgaaag 180

aagccaacac taaaccacaa atatacaaca aggccatttt ctcaaacgag agtcagcctt 240

taacgaaatg accatggttg acacagagat gccattctgg cccaccaact ttgggatcag 300

ctccgtggat ctctccgtaa tggaagacca ctcccactcc tttgatatca agcccttcac 360

tactgttgac ttctccagca tttctactcc acattacgaa gacattccat tcacaagaac 420

agatccagtg gttgcagatt acaagtatga cctgaaactt caagagtacc aaagtgcaat 480

caaagtggag cctgcatctc caccttatta ttctgagaag actcagctct acaataagcc 540

tcatgaagag ccttccaact ccctcatggc aattgaatgt cgtgtctgtg gagataaagc 600

ttctggattt cactatggag ttcatgcttg tgaaggatgc aagggtttct tccggagaac 660

aatcagattg aagcttatct atgacagatg tgatcttaac tgtcggatcc acaaaaaaag 720

tagaaataaa tgtcagtact gtcggtttca gaaatgcctt gcagtgggga tgtctcataa 780

tgccatcagg tttgggcgga tgccacaggc cgagaaggag aagctgttgg cggagatctc 840

cagtgatatc gaccagctga atccagagtc cgctgacctc cgggccctgg caaaacattt 900

gtatgactca tacataaagt ccttcccgct gaccaaagca aaggcgaggg cgatcttgac 960

aggaaagaca acagacaaat caccattcgt tatctatgac atgaattcct taatgatggg 1020

agaagataaa atcaagttca aacacatcac ccccctgcag gagcagagca aagaggtggc 1080

catccgcatc tttcagggct gccagtttcg ctccgtggag gctgtgcagg agatcacaga 1140

gtatgccaaa agcattcctg gttttgtaaa tcttgacttg aacgaccaag taactctcct 1200

caaatatgga gtccacgaga tcatttacac aatgctggcc tccttgatga ataaagatgg 1260

ggttctcata tccgagggcc aaggcttcat gacaagggag tttctaaaga gcctgcgaaa 1320

gccttttggt gactttatgg agcccaagtt tgagtttgct gtgaagttca atgcactgga 1380

attagatgac agcgacttgg caatatttat tgctgtcatt attctcagtg gagaccgccc 1440

aggtttgctg aatgtgaagc ccattgaaga cattcaagac aacctgctac aagccctgga 1500

gctccagctg aagctgaacc accctgagtc ctcacagctg tttgccaagc tgctccagaa 1560

aatgacagac ctcagacaga ttgtcacgga acacgtgcag ctactgcagg tgatcaagaa 1620

gacggagaca gacatgagtc ttcacccgct cctgcaggag atctacaagg acttgtacta 1680

gcagagagtc ctgagccact gccaacattt cccttcttcc agttgcacta ttctgaggga 1740

aaatctgaca cctaagaaat ttactgtgaa aaagcatttt aaaaagaaaa ggttttagaa 1800

tatgatctat tttatgcata ttgtttataa agacacattt acaatttact tttaatatta 1860

aaaattacca tattatgaaa ttgctgatag ta 1892

<210> 5
<211> 2113
<212> DNA
<213> Homo sapiens

<400> 5
tcccaatccc ggggcggccg ggcgggggtg ggcagggggc gtgaggccgc ccctgcgtcc 60

cgggggcccc ccgaaaacgc gctccgggtg cccggtccct ccgctgcgcc ctgccgccgt 120

cctcccgggg gtctcgggcg gccgcggccg tgtccttcgc gtcccggcgg cgcggcggga 180

ggggccggcg tgacgcagcg gttgctacgg gccgccctta taaataaccg ggctcaggag 240

aaactttagc gagtcagagc cgcgcacggg actgggaagg ggacccaccc gagggtccag 300

ccaccagccc cctcactaat agcggccacc ccggcagcgg cggcagcagc agcagcgacg 360

cagcggcgac agctcagagc agggaggccg cgccacctgc gggccggccg gagcgggcag 420

ccccaggccc cctccccggg cacccgcgtt catgcaacgc ctggtggcct gggacccagc 480

atgtctcccc ctgccgccgc cgccgcctgc ctttaaatcc atggaagtgg ccaacttcta 540

ctacgaggcg gactgcttgg ctgctgcgta cggcggcaag gcggcccccg cggcgccccc 600

cgcggccaga cccgggccgc gcccccccgc cggcgagctg ggcagcatcg gcgaccacga 660

gcgcgccatc gacttcagcc cgtacctgga gccgctgggc gcgccgcagg ccccggcgcc 720

cgccacggcc acggacacct tcgaggcggc tccgcccgcg cccgcccccg cgcccgcctc 780

ctccgggcag caccacgact tcctctccga cctcttctcc gacgactacg ggggcaagaa 840

ctgcaagaag ccggccgagt acggctacgt gagcctgggg cgcctggggg ccgccaaggg 900

cgcgctgcac cccggctgct tcgcgcccct gcacccaccg cccccgccgc cgccgccgcc 960

cgccgagctc aaggcggagc cgggcttcga gcccgcggac tgcaagcgga aggaggaggc 1020

cggggcgccg ggcggcggcg caggcatggc ggcgggcttc ccgtacgcgc tgcgcgctta 1080

cctcggctac caggcggtgc cgagcggcag cagcgggagc ctctccacgt cctcctcgtc 1140

cagcccgccc ggcacgccga gccccgctga cgccaaggcg cccccgaccg cctgctacgc 1200

gggggccgcg ccggcgccct cgcaggtcaa gagcaaggcc aagaagaccg tggacaagca 1260

cagcgacgag tacaagatcc ggcgcgagcg caacaacatc gccgtgcgca agagccgcga 1320

caaggccaag atgcgcaacc tggagacgca gcacaaggtc ctggagctca cggccgagaa 1380

cgagcggctg cagaagaagg tggagcagct gtcgcgcgag ctcagcaccc tgcggaactt 1440

gttcaagcag ctgcccgagc ccctgctcgc ctcctccggc cactgctagc gcggcccccg 1500

cgcgcgtccc cctgccggcc ggggctgaga ctccggggag cgcccgcgcc cgcgccctcg 1560

cccccgcccc cggcggcgcc ggcaaaactt tggcactggg gcacttggca gcgcggggag 1620

cccgtcggta attttaatat tttattatat atatatatct atatttttgt ccaaaccaac 1680

cgcacatgca gatggggctc ccgcccgtgg tgttatttaa agaagaaacg tctatgtgta 1740

cagatgaatg ataaactctc tgcttctccc tctgcccctc tccaggcgcc ggcgggcggg 1800

ccggtttcga agttgatgca atcggtttaa acatggctga acgcgtgtgt acacgggact 1860

gacgcaaccc acgtgtaact gtcagccggg ccctgagtaa tcgcttaaag atgttcctac 1920

gggcttgttg ctgttgatgt tttgttttgt tttgtttttt ggtctttttt tgtattataa 1980

aaaataatct atttctatga gaaaagaggc gtctgtatat tttgggaatc ttttccgttt 2040

caagcattaa gaacactttt aataaacttt tttttgagaa tggttacaaa gccttttggg 2100

ggcagtaaaa aaa 2113

<210> 6
<211> 3444
<212> DNA
<213> Homo sapiens

<400> 6
gtaggaatcg cagcgccagc ggttgcaagg cccaagaagc ccatcctggg aaggaaaatg 60

cattggggaa ccctgtgcgg attcttgtgg ctttggccct atcttttcta tgtccaagct 120

gtgcccatcc aaaaagtcca agatgacacc aaaaccctca tcaagacaat tgtcaccagg 180

atcaatgaca tttcacacac gcagtcagtc tcctccaaac agaaagtcac cggtttggac 240

ttcattcctg ggctccaccc catcctgacc ttatccaaga tggaccagac actggcagtc 300

taccaacaga tcctcaccag tatgccttcc agaaacgtga tccaaatatc caacgacctg 360

gagaacctcc gggatcttct tcacgtgctg gccttctcta agagctgcca cttgccctgg 420

gccagtggcc tggagacctt ggacagcctg gggggtgtcc tggaagcttc aggctactcc 480

acagaggtgg tggccctgag caggctgcag gggtctctgc aggacatgct gtggcagctg 540

gacctcagcc ctgggtgctg aggccttgaa ggtcactctt cctgcaagga ctacgttaag 600

ggaaggaact ctggcttcca ggtatctcca ggattgaaga gcattgcatg gacacccctt 660

atccaggact ctgtcaattt ccctgactcc tctaagccac tcttccaaag gcataagacc 720

ctaagcctcc ttttgcttga aaccaaagat atatacacag gatcctattc tcaccaggaa 780

gggggtccac ccagcaaaga gtgggctgca tctgggattc ccaccaaggt cttcagccat 840

caacaagagt tgtcttgtcc cctcttgacc catctccccc tcactgaatg cctcaatgtg 900

accaggggtg atttcagaga gggcagaggg gtaggcagag cctttggatg accagaacaa 960

ggttccctct gagaattcca aggagttcca tgaagaccac atccacacac gcaggaactc 1020

ccagcaacac aagctggaag cacatgttta tttattctgc attttattct ggatggattt 1080

gaagcaaagc accagcttct ccaggctctt tggggtcagc cagggccagg ggtctccctg 1140

gagtgcagtt tccaatccca tagatgggtc tggctgagct gaacccattt tgagtgactc 1200

gagggttggg ttcatctgag caagagctgg caaaggtggc tctccagtta gttctctcgt 1260

aactggtttc atttctactg tgactgatgt tacatcacag tgtttgcaat ggtgttgccc 1320

tgagtggatc tccaaggacc aggttatttt aaaaagattt gttttgtcaa gtgtcatatg 1380

taggtgtctg cacccagggg tggggaatgt ttgggcagaa gggagaagga tctagaatgt 1440

gttttctgaa taacatttgt gtggtgggtt ctttggaagg agtgagatca ttttcttatc 1500

ttctgcaatt gcttaggatg tttttcatga aaatagctct ttcagggggg ttgtgaggcc 1560

tggccaggca ccccctggag agaagtttct ggccctggct gaccccaaag agcctggaga 1620

agctgatgct ttgcttcaaa tccatccaga ataaaacgca aagggctgaa agccatttgt 1680

tggggcagtg gtaagctctg gctttctccg actgctaggg agtggtcttt cctatcatgg 1740

agtgacggtc ccacactggt gactgcgatc ttcagagcag gggtccttgg tgtgaccctc 1800

tgaatggtcc agggttgatc acactctggg tttattacat ggcagtgttc ctatttgggg 1860

cttgcatgcc aaattgtagt tcttgtctga ttggctcacc caagcaaggc caaaattacc 1920

aaaaatcttg gggggttttt actccagtgg tgaagaaaac tcctttagca ggtggtcctg 1980

agacctgaca agcactgcta ggcgagtgcc aggactcccc aggccaggcc accaggatgg 2040

cccttcccac tggaggtcac attcaggaag atgaaagagg aggtttgggg tctgccacca 2100

tcctgctgct gtgtttttgc tatcacacag tgggtggtgg atctgtccaa ggaaacttga 2160

atcaaagcag ttaactttaa gactgagcac ctgcttcatg ctcagccctg actggtgcta 2220

taggctggag aagctcaccc aataaacatt aagattgagg cctgccctca gggatcttgc 2280

attcccagtg gtcaaaccgc actcacccat gtgccaaggt ggggtattta ccacagcagc 2340

tgaacagcca aatgcatggt gcagttgaca gcaggtggga aatggtatga gctgaggggg 2400

gccgtgccca ggggcccaca gggaaccctg cttgcacttt gtaacatgtt tacttttcag 2460

ggcatcttag cttctattat agccacatcc ctttgaaaca agataactga gaatttaaaa 2520

ataagaaaat acataagacc ataacagcca acaggtggca ggaccaggac tatagcccag 2580

gtcctctgat acccagagca ttacgtgagc caggtaatga gggactggaa ccagggagac 2640

cgagcgcttt ctggaaaaga ggagtttcga ggtagagttt gaaggaggtg agggatgtga 2700

attgcctgca gagagaagcc tgttttgttg gaaggtttgg tgtgtggaga tgcagaggta 2760

aaagtgtgag cagtgagtta cagcgagagg cagagaaaga agagacagga gggcaagggc 2820

catgctgaag ggaccttgaa gggtaaagaa gtttgatatt aaaggagtta agagtagcaa 2880

gttctagaga agaggctggt gctgtggcca gggtgagagc tgctctggaa aatgtgaccc 2940

agatcctcac aaccacctaa tcaggctgag gtgtcttaag ccttttgctc acaaaacctg 3000

gcacaatggc taattcccag agtgtgaaac ttcctaagta taaatggttg tctgtttttg 3060

taacttaaaa aaaaaaaaaa aagtttggcc gggtgcggtg gctcacgcct gtaatcccag 3120

cactttggga ggccaaggtg gggggatcac aaggtcacta gatggcgagc atcctggcca 3180

acatggtgaa accccgtctc tactaaaaac acaaaagtta gctgagcgtg gtggcgggcg 3240

cctgtagtcc cagccactcg ggaggctgag acaggagaat cgcttaaacc tgggaggcgg 3300

agagtacagt gagccaagat cgcgccactg cactccggcc tgatgacaga gcgagattcc 3360

gtcttaaaaa aaaaaaaaaa aaagtttgtt tttaaaaaaa tctaaataaa ataactttgc 3420

cccctgcaaa aaaaaaaaaa aaaa 3444

<210> 7
<211> 4629
<212> DNA
<213> Homo sapiens

<400> 7
aggctgttga ggctgggcca tctcctcctc acttccattc tgactgcagt ctgtggttct 60

gattccatac cagaggagac gggatttcac catgttgtcc aggctggtct gaaactcctg 120

acatcagggc tcaggatgct gttgctggga gctgttctac tgctattagc tctgcccggt 180

catgaccagg aaaccacgac tcaagggccc ggagtcctgc ttcccctgcc caagggggcc 240

tgcacaggtt ggatggcggg catcccaggg catccgggcc ataatggggc cccaggccgt 300

gatggcagag atggcacccc tggtgagaag ggtgagaaag gagatccagg tcttattggt 360

cctaagggag acatcggtga aaccggagta cccggggctg aaggtccccg aggctttccg 420

ggaatccaag gcaggaaagg agaacctgga gaaggtgcct atgtataccg ctcagcattc 480

agtgtgggat tggagactta cgttactatc cccaacatgc ccattcgctt taccaagatc 540

ttctacaatc agcaaaacca ctatgatggc tccactggta aattccactg caacattcct 600

gggctgtact actttgccta ccacatcaca gtctatatga aggatgtgaa ggtcagcctc 660

ttcaagaagg acaaggctat gctcttcacc tatgatcagt accaggaaaa taatgtggac 720

caggcctccg gctctgtgct cctgcatctg gaggtgggcg accaagtctg gctccaggtg 780

tatggggaag gagagcgtaa tggactctat gctgataatg acaatgactc caccttcaca 840

ggctttcttc tctaccatga caccaactga tcaccactaa ctcagagcct cctccaggcc 900

aaacagcccc aaagtcaatt aaaggctttc agtacggtta ggaagttgat tattatttag 960

ttggaggcct ttagatatta ttcattcatt tactcattca tttattcatt cattcatcga 1020

gtaactttaa aaaaatcata tgctatgttc ccagtcctgg ggagcttcac aaacatgacc 1080

agataactga ctagaaagaa gtagttgaca gtgctatttt gtgcccactg tctctcctga 1140

tgctcatatc aatcctataa ggcacaggga acaagcattc tcctgttttt acagattgta 1200

tcctgaggct gagagagtta agtgaatgtc taaggtcaca cagtattaag tgacagtgct 1260

agaaatcaaa cccagagctg tggactttgt tcactagact gtgccctttt atagaggtac 1320

atgttctctt tggagtgttg gtaggtgtct gtttcccacc tcacctgaga gccattgaat 1380

ttgccttcct catgaattaa aacctccccc aagcagagct tcctcagaga aagtggttct 1440

atgatgacgt cctgtcttgg aaggactact actcaatggc ccctgcacta ctctacttcc 1500

tcttacctat gtcccttctc atgcctttcc ctccaacggg gaaagccaac tccatctcta 1560

agtgccgaac tcatccctgt tcctcaaggc cacctggcca ggagcttctc tgatgtgata 1620

tccacttttt ttttttttga gatggagtct cactctgtca cccaggctgg agtacagtga 1680

cacgacctcg gctcactgca gcctccttct cctgggtcca agcaattatt gtgcctcagc 1740

ctcccgagta gctgagactt caggtgcatt ccaccacaca tggctaattt ttgtattttt 1800

agtagaaatg gggtttcgtc atgttggcca ggctggtctc gaactcctgg cctaggtgat 1860

ccacccgcct cgacctccca aagtgctggg attacaggca tgagccacca tgcccagtcg 1920

atatctcact tttatttttg ccatggatga gagtcctggg tgtgaggaac acctcccacc 1980

aggctagagg caactgccca ggaaggactg tgcttccgtc acctctaaat cccttgcaga 2040

tccttgataa atgcctcatg aagaccaatc tcttgaatcc catatctacc cagaattaac 2100

tccattccag tctctgcatg taatcagttt tatccacaga aacattttca ttttaggaaa 2160

tccctggttt taagtatcaa tccttgttca gctggacaat atgaatcttt tccactgaag 2220

ttagggatga ctgtgatttt cagaacacgt ccagaatttt tcatcaagaa ggtagcttga 2280

gcctgaaatg caaaacccat ggaggaattc tgaagccatt gtctccttga gtaccaacag 2340

ggtcagggaa gactgggcct cctgaattta ttattgttct ttaagaatta caggttgagg 2400

tagttgatgg tggtaaacat tctctcagga gacaataact ccagtgatgt tcttcaaaga 2460

ttttagcaaa aacagagtaa atagcattct ctatcaatat ataaatttaa aaaactatct 2520

ttttgcttac agttttaaat tctgaacaat tctctcttat atgtgtattg ctaatcatta 2580

aggtattatt ttttccacat ataaagcttt gtctttttgt tgttgttgtt gtttttaaga 2640

tggagtttcc ctctgttgcc aggctagagt gcagtggcat gatctcggct tactgcaacc 2700

tttgcctccc aggttcaagc gattcttctg cctcagcctc ccgagtagct gggaccacag 2760

gtgcctacca ccatgccagg ctaatttttg tatttttagt aaagacaggg tttcaccata 2820

ttggccaggc tggtctcgaa ctcctgacct tgtgatctgc ccgcctccat ttttgttgtt 2880

attttttgag aaagatagat atgaggttta gagagggatg aagaggtgag agtaagcctt 2940

gtgttagtca gaactctgtg ttgtgaatgt cattcacaac agaaaaccca aaatattatg 3000

caaactactg taagcaagaa aaataaagga aaaatggaaa catttattcc tttgcataat 3060

agaaattacc agagttgttc tgtctttaga taaggtttga accaaagctc aaaacaatca 3120

agaccctttt ctgtatgtcc ttctgttctg ccttccgcag tgtaggcttt accctcaggt 3180

gctacacagt atagttctag ggtttccctc ccgatatcaa aaagactgtg gcctgcccag 3240

ctctcgtatc cccaagccac accatctggc taaatggaca tcatgttttc tggtgatgcc 3300

caaagaggag agaggaagct ctctttccca gatgccccag caagtgtaac cttgcatctc 3360

attgctctgg ctgagttgtg tgcctgtttc tgaccaatca ctgagtcagg aggatgaaat 3420

attcatattg acttaattgc agcttaagtt aggggtatgt agaggtattt tccctaaagc 3480

aaaattggga cactgttatc agaaatagga gagtggatga tagatgcaaa ataatacctg 3540

tccacaacaa actcttaatg ctgtgtttga gctttcatga gtttcccaga gagacatagc 3600

tggaaaattc ctattgattt tctctaaaat ttcaacaagt agctaaagtc tggctatgct 3660

cacagtctca catctggttg gggtgggctc cttacagaac acgctttcac agttacccta 3720

aactctctgg ggcagggtta ttcctttgtg gaaccagagg cacagagaga gtcaactgag 3780

gccaaaagag gcctgagaga aactgaggtc aagatttcag gattaatggt cctgtgatgc 3840

tttgaagtac aattgtggat ttgtccaatt ctctttagtt ctgtcagctt ttgcttcata 3900

tattttagcg ctctattatt agatatatac atgtttagta ttatgtctta ttggtgcatt 3960

tactctctta tcattatgta atgtccttct ttatctgtga taattttctg tgttctgaag 4020

tctactttgt ctaaaaataa catacgcact caacttcctt ttctttcttc cttcctttct 4080

ttcttccttc ctttctttct ctctctctct ctttccttcc ttccttcctc cttttctttc 4140

tctctctctc tctctctctt tttttgacag actctcgttc tgtggccctg gctggagttc 4200

agtggtgtga tcttggctca ctgctacctc taccatgagc aattctcctg cctcagcctc 4260

ccaagtagct ggaactacag gctcatgcca ctgcgcccag ctaatttttg tatttttcgt 4320

agagacgggg tttcaccaca ttcgtcaggt tggtttcaaa ctcctgactt tgtgatccac 4380

ccgcctcggc ctcccaaagt gctgggatta caggcatgag ccatcacacc tggtcaactt 4440

tcttttgatt agtgtttttg tggtatatct ttttccatca tgttacttta aatatatcta 4500

tattattgta tttaaaatgt gtttcttaca gactgcatgt agttgggtat aatttttatc 4560

cagtctaaaa atatctgtct tttaattggt gtttagacaa tttatattta ataaaattgt 4620

tgaatttaa 4629

<210> 8
<211> 4578
<212> DNA
<213> Homo sapiens

<400> 8
aggctgttga ggctgggcca tctcctcctc acttccattc tgactgcagt ctgtggttct 60

gattccatac cagaggggct caggatgctg ttgctgggag ctgttctact gctattagct 120

ctgcccggtc atgaccagga aaccacgact caagggcccg gagtcctgct tcccctgccc 180

aagggggcct gcacaggttg gatggcgggc atcccagggc atccgggcca taatggggcc 240

ccaggccgtg atggcagaga tggcacccct ggtgagaagg gtgagaaagg agatccaggt 300

cttattggtc ctaagggaga catcggtgaa accggagtac ccggggctga aggtccccga 360

ggctttccgg gaatccaagg caggaaagga gaacctggag aaggtgccta tgtataccgc 420

tcagcattca gtgtgggatt ggagacttac gttactatcc ccaacatgcc cattcgcttt 480

accaagatct tctacaatca gcaaaaccac tatgatggct ccactggtaa attccactgc 540

aacattcctg ggctgtacta ctttgcctac cacatcacag tctatatgaa ggatgtgaag 600

gtcagcctct tcaagaagga caaggctatg ctcttcacct atgatcagta ccaggaaaat 660

aatgtggacc aggcctccgg ctctgtgctc ctgcatctgg aggtgggcga ccaagtctgg 720

ctccaggtgt atggggaagg agagcgtaat ggactctatg ctgataatga caatgactcc 780

accttcacag gctttcttct ctaccatgac accaactgat caccactaac tcagagcctc 840

ctccaggcca aacagcccca aagtcaatta aaggctttca gtacggttag gaagttgatt 900

attatttagt tggaggcctt tagatattat tcattcattt actcattcat ttattcattc 960

attcatcgag taactttaaa aaaatcatat gctatgttcc cagtcctggg gagcttcaca 1020

aacatgacca gataactgac tagaaagaag tagttgacag tgctattttg tgcccactgt 1080

ctctcctgat gctcatatca atcctataag gcacagggaa caagcattct cctgttttta 1140

cagattgtat cctgaggctg agagagttaa gtgaatgtct aaggtcacac agtattaagt 1200

gacagtgcta gaaatcaaac ccagagctgt ggactttgtt cactagactg tgccctttta 1260

tagaggtaca tgttctcttt ggagtgttgg taggtgtctg tttcccacct cacctgagag 1320

ccattgaatt tgccttcctc atgaattaaa acctccccca agcagagctt cctcagagaa 1380

agtggttcta tgatgacgtc ctgtcttgga aggactacta ctcaatggcc cctgcactac 1440

tctacttcct cttacctatg tcccttctca tgcctttccc tccaacgggg aaagccaact 1500

ccatctctaa gtgccgaact catccctgtt cctcaaggcc acctggccag gagcttctct 1560

gatgtgatat ccactttttt tttttttgag atggagtctc actctgtcac ccaggctgga 1620

gtacagtgac acgacctcgg ctcactgcag cctccttctc ctgggtccaa gcaattattg 1680

tgcctcagcc tcccgagtag ctgagacttc aggtgcattc caccacacat ggctaatttt 1740

tgtattttta gtagaaatgg ggtttcgtca tgttggccag gctggtctcg aactcctggc 1800

ctaggtgatc cacccgcctc gacctcccaa agtgctggga ttacaggcat gagccaccat 1860

gcccagtcga tatctcactt tttattttgc catggatgag agtcctgggt gtgaggaaca 1920

cctcccacca ggctagaggc aactgcccag gaaggactgt gcttccgtca cctctaaatc 1980

ccttgcagat ccttgataaa tgcctcatga agaccaatct cttgaatccc atatctaccc 2040

agaattaact ccattccagt ctctgcatgt aatcagtttt atccacagaa acattttcat 2100

tttaggaaat ccctggtttt aagtatcaat ccttgttcag ctggacaata tgaatctttt 2160

ccactgaagt tagggatgac tgtgattttc agaacacgtc cagaattttt catcaagaag 2220

gtagcttgag cctgaaatgc aaaacccatg gaggaattct gaagccattg tctccttgag 2280

taccaacagg gtcagggaag actgggcctc ctgaatttat tattgttctt taagaattac 2340

aggttgaggt agttgatggt ggtaaacatt ctctcaggag acaataactc cagtgatgtt 2400

cttcaaagat tttagcaaaa acagagtaaa tagcattctc tatcaatata taaatttaaa 2460

aaactatctt tttgcttaca gttttaaatt ctgaacaatt ctctcttata tgtgtattgc 2520

taatcattaa ggtattattt tttccacata taaagctttg tctttttgtt gttgttgttg 2580

tttttaagat ggagtttccc tctgttgcca ggctagagtg cagtggcatg atctcggctt 2640

actgcaacct ttgcctccca ggttcaagcg attcttctgc ctcagcctcc cgagtagctg 2700

ggaccacagg tgcctaccac catgccaggc taatttttgt atttttagta aagacagggt 2760

ttcaccatat tggccaggct ggtctcgaac tcctgacctt gtgatctgcc cgcctccatt 2820

tttgttgtta ttttttgaga aagatagata tgaggtttag agagggatga agaggtgaga 2880

gtaagccttg tgttagtcag aactctgtgt tgtgaatgtc attcacaaca gaaaacccaa 2940

aatattatgc aaactactgt aagcaagaaa aataaaggaa aaatggaaac atttattcct 3000

ttgcataata gaaattacca gagttgttct gtctttagat aaggtttgaa ccaaagctca 3060

aaacaatcaa gacccttttc tgtatgtcct tctgttctgc cttccgcagt gtaggcttta 3120

ccctcaggtg ctacacagta tagttctagg gtttccctcc cgatatcaaa aagactgtgg 3180

cctgcccagc tctcgtatcc ccaagccaca ccatctggct aaatggacat catgttttct 3240

ggtgatgccc aaagaggaga gaggaagctc tctttcccag atgccccagc aagtgtaacc 3300

ttgcatctca ttgctctggc tgagttgtgt gcctgtttct gaccaatcac tgagtcagga 3360

ggatgaaata ttcatattga cttaattgca gcttaagtta ggggtatgta gaggtatttt 3420

ccctaaagca aaattgggac actgttatca gaaataggag agtggatgat agatgcaaaa 3480

taatacctgt ccacaacaaa ctcttaatgc tgtgtttgag ctttcatgag tttcccagag 3540

agacatagct ggaaaattcc tattgatttt ctctaaaatt tcaacaagta gctaaagtct 3600

ggctatgctc acagtctcac atctggttgg ggtgggctcc ttacagaaca cgctttcaca 3660

gttaccctaa actctctggg gcagggttat tcctttgtgg aaccagaggc acagagagag 3720

tcaactgagg ccaaaagagg cctgagagaa actgaggtca agatttcagg attaatggtc 3780

ctgtgatgct ttgaagtaca attgtggatt tgtccaattc tctttagttc tgtcagcttt 3840

tgcttcatat attttagcgc tctattatta gatatataca tgtttagtat tatgtcttat 3900

tggtgcattt actctcttat cattatgtaa tgtccttctt tatctgtgat aattttctgt 3960

gttctgaagt ctactttgtc taaaaataac atacgcactc aacttccttt tctttcttcc 4020

ttcctttctt tcttccttcc tttctttctc tctctctctc tttccttcct tccttcctcc 4080

ttttctttct ctctctctct ctctctcttt ttttgacaga ctctcgttct gtggccctgg 4140

ctggagttca gtggtgtgat cttggctcac tgctacctct accatgagca attctcctgc 4200

ctcagcctcc caagtagctg gaactacagg ctcatgccac tgcgcccagc taatttttgt 4260

atttttcgta gagacggggt ttcaccacat tcgtcaggtt ggtttcaaac tcctgacttt 4320

gtgatccacc cgcctcggcc tcccaaagtg ctgggattac aggcatgagc catcacacct 4380

ggtcaacttt cttttgatta gtgtttttgt ggtatatctt tttccatcat gttactttaa 4440

atatatctat attattgtat ttaaaatgtg tttcttacag actgcatgta gttgggtata 4500

atttttatcc agtctaaaaa tatctgtctt ttaattggtg tttagacaat ttatatttaa 4560

taaaattgtt gaatttaa 4578

## Description

### FIELD AND BACKGROUND OF THE INVENTION

The present invention, in some embodiments thereof, relates to devices for surgical applications and to methods of using same for various reconstructive surgeries.

The aim of regenerative medicine is to repair or replace damaged or diseased tissue in the human body.

The challenge in any reconstructive procedure is to provide a supporting structure while restoring the normal anatomic condition of the surrounding tissues. Though several materials can potentially provide the mechanical support, they do not possess the properties necessary to restore the living tissue's original quality.

Abdominal ventral hernia and pelvic floor defect (PFD) are common and challenging conditions for surgeons. It is estimated that 250,000 hernia repair and 300,000 procedures of prolapse and urinary incontinence surgeries are performed each year in the US. However, in about 12.5% of the hernia repair and 29% of the pelvic prolapse repairs repeated surgeries are needed within 5 years of initial surgery, mainly due to infection, seroma, wound dehiscence and formation of enterocutaneous fistula.

Synthetic meshes made of polypropylene and polyester are used for reconstructive surgeries [e.g., the Gyncare Prolift® (Ethicon, Johnson & Johnson, USA]. Although the synthetic meshes are available and can simplify the operative procedure, reduce patient discomfort from an additional incision site and decrease operative time, in about 2.8-17.3% of the cases these meshes cause foreign-body reaction with risks of infection, rejection, visceral adhesion to the repair site, erosion to the bowel, urinary bladder and vaginal mucosa leading to enterocutaneous fistula, bowel obstruction and urinary bladder complications, extrusion of the repair material and infection. Infected synthetic repair material often necessitates surgical removal, leaving a contaminated field and a hernia deficit larger than the original (van't Riet M, et al., 2007. Hernia. 11:409-13; de Vries Reilingh TS, et al., 2007, World J Surg. 31:756-63).

Additional background art includes U.S. Patent Application No. 20100185219 (to Arthur A. Gertzman et al.), and WO 2012/014205 which is hereby incorporated by reference in its entirety.

### SUMMARY OF THE INVENTION

According to an aspect of some embodiments of the present invention there is provided a device comprising at least two layers, the at least two layers being at least partially overlapping (e.g., superposed) and contacting one another, wherein a first layer of the at least two layers comprises a mesh, and wherein a second layer of the at least two layers comprises an electrospun element, and wherein the device is devoid of an extracellular matrix generated by mesenchymal progenitor cells, which are characterized by a reduced differentiation potential into an adipogenic lineage by at least about 50% as compared to differentiation potential of mesenchymal stem cells from an adult adipose source under identical assay conditions, and by an increased osteogenic differentiation potential by at least about 20% as compared to the osteogenic differentiation potential of adipose-derived MSCs under identical assays conditions.

According to some embodiments of the invention, the device further comprises extracellular matrix (ECM), with the proviso that the ECM is not generated by mesenchymal progenitor cells, which are characterized by a reduced differentiation potential into an adipogenic lineage by at least about 50% as compared to differentiation potential of mesenchymal stem cells from an adult adipose source under identical assay conditions, and by an increased osteogenic differentiation potential by at least about 20% as compared to the osteogenic differentiation potential of adipose-derived MSCs under identical assays conditions.

According to an aspect of some embodiments of the present invention there is provided a method of generating the device of some embodiments of the invention, comprising electrospinning a polymeric solution onto a mesh, thereby obtaining a layer of an electrospun element over a layer of the mesh, thereby generating the device.

According to an aspect of some embodiments of the present invention there is provided a method of treating a subject in need of a reconstructive surgery, comprising implanting the device of some embodiments of the invention in the subject in a manner suitable for reconstructing a tissue or an organ of the subject, thereby treating the subject in need of the reconstructive surgery.

According to an aspect of some embodiments of the present invention there is provided a use of the device of some embodiments of the invention as suburethral sling.

According to some embodiments of the invention, the electrospun element adheres to the mesh by physical forces.

According to some embodiments of the invention, the mesh is non-biodegradable.

According to some embodiments of the invention, the mesh is biodegradable.

According to some embodiments of the invention, the mesh is partially biodegradable.

According to some embodiments of the invention, the mesh comprises a biocompatible material.

According to some embodiments of the invention, the electrospun element comprises a biocompatible polymer.

According to some embodiments of the invention, the electrospun element is biodegradable.

According to some embodiments of the invention, the mesh is made of a woven material.

According to some embodiments of the invention, the mesh is made of a nonwoven material.

According to some embodiments of the invention, the mesh is made of a metal or a polymer.

According to some embodiments of the invention, the electrospun element comprises a nonwoven nanofiber.

According to some embodiments of the invention, the electrospun element comprises oriented fibers.

According to some embodiments of the invention, the electrospun element comprises non-oriented fibers.

According to some embodiments of the invention, the first layer and the second layer are connected to each other by non-covalent bonds.

According to some embodiments of the invention, the electrospun element comprises an active ingredient attached thereto.

According to some embodiments of the invention, the subject suffers from a pathology selected from the group consisting of abdominal ventral hernia, inguinal hernia, diaphragmatic hernia, pelvic floor defect (PFD), pelvic organ prolapse, and stress urinary incontinence.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

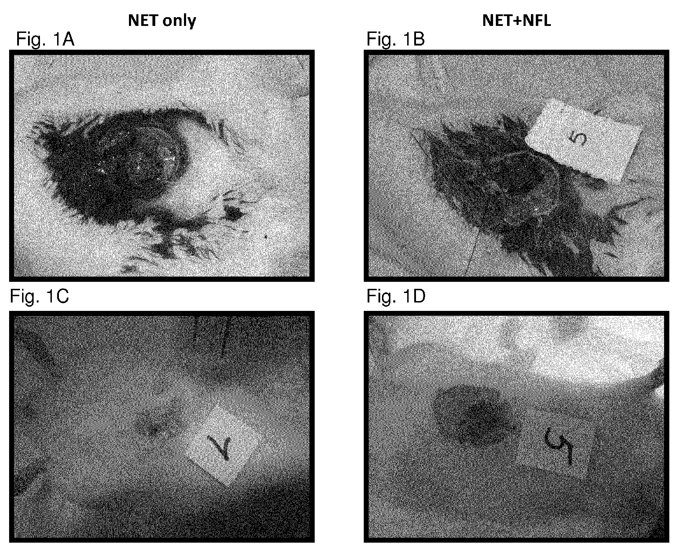

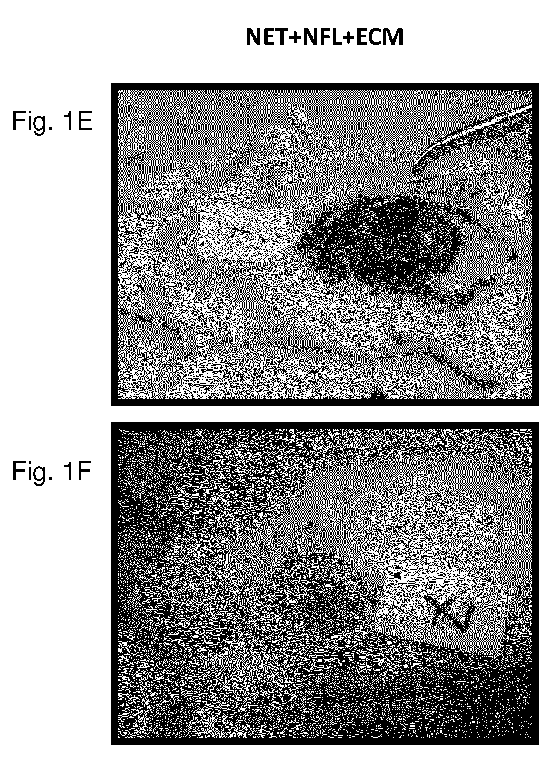

FIGs. 1A-F are photographs of rats subjected to iatrogenic abdominal hernia following implantation of various devices. Figures 1A, B and E - devices during the implantation to rats. Figures 1C, D and F - devices after 8 weeks at euthanization. The implants included: (1) Net (mesh) only - Prolift™ (Figures 1A and 1C); (2) Net+NFL device, i.e., a Prolift™ mesh coated with electrospun element (NFL) composed PCL:PLGA at a ratio of 1:6 (weight ratio) (Figures 1B and 1D); and (3) Net+NFL+ECM device, *i.e.,* a Prolift™ mesh coated with electrospun element (NFL) composed PCL:PLGA at a ratio of 1:6 (w/w), with acellular AD5T derived ECM (Figures 1E and 1F).

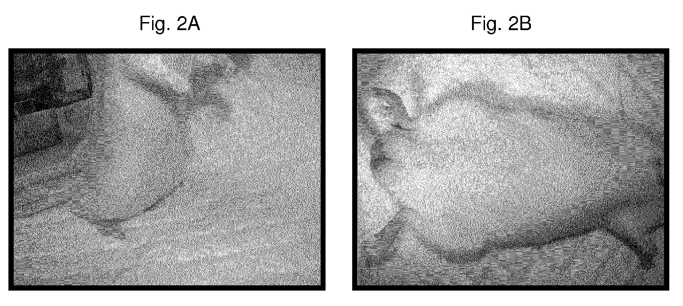

FIGs. 2A-B are photographs taken 8 weeks following implantation depicting external recovery of the incision with no dehiscence of the abdominal scar in all the animals.

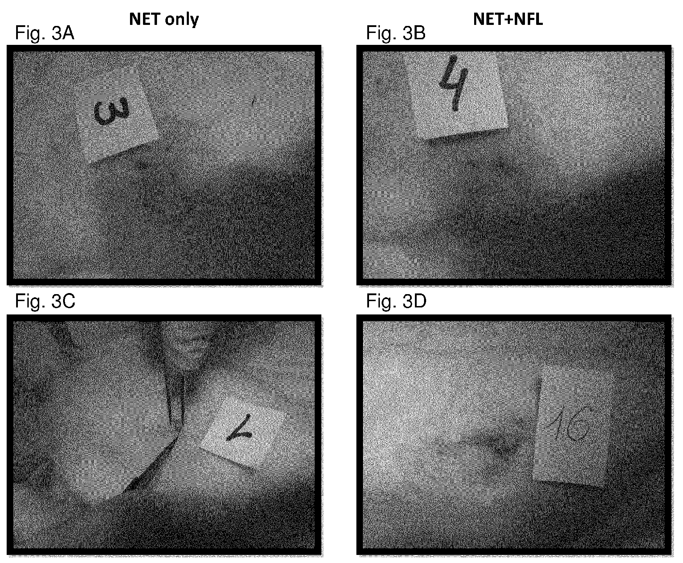

FIGs. 3A-D are photographs depicting erosion of the implant through the abdominal scar in rats implanted with the Net (mesh) only (Figures 3A and 3C) or with the Net (mesh) + NFL (electrospun element) device (Figures 3B and 3D). It should be noted that such erosion of the implant was observed in about 50%-60% of the animals implanted with the mesh alone and in about 40% of the animals implanted with the mesh + electrospun element (Net + NFL) implant (device), but not in the rats implanted with Net+NFL+ECM device (data not shown).

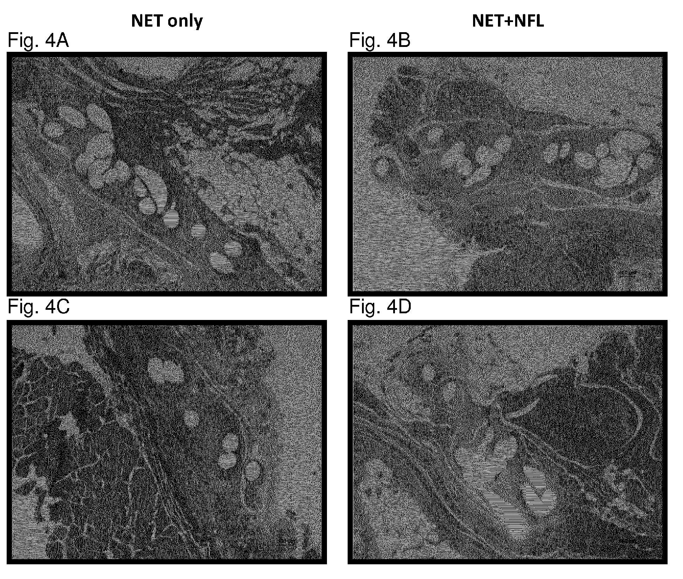

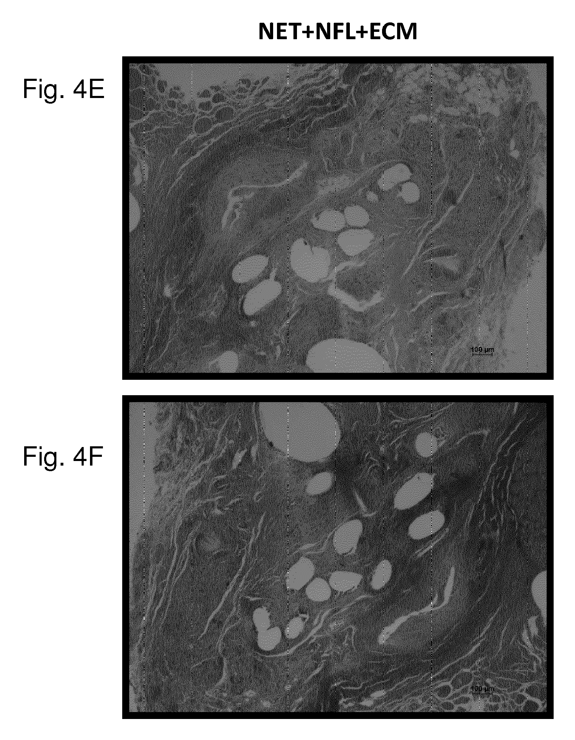

FIGs. 4A-F are histological analyses of the implants with the adjacent tissues. At sacrifice, the implants were carefully removed from adjacent tissues and examined by histological analysis of tissue sections stained with Masson's Trichrome (TC) identifying matrix collagens. Figures 4A and 4C - implants made of mesh (net) alone. Figures 4B and 4D - implants made of mesh + electrospun element (Net + NFL). Figures 4E and 4F - implants made of mesh + electrospun element + extracellular matrix (Net + NFL + ECM). The areas stained in blue represent the newly formed collagen around the mesh. The mesh is shown as relatively big lacunas. Macrophages polymorphonuclear cells and small blood vessels can also identified around the mesh area. In order to quantify the amount of newly formed collagen and to compare its quantity between the different types of implants (e.g., meshes alone, or with the NFL, and/or with ECM), which were used in the experiment, a computerized morphometry was performed.

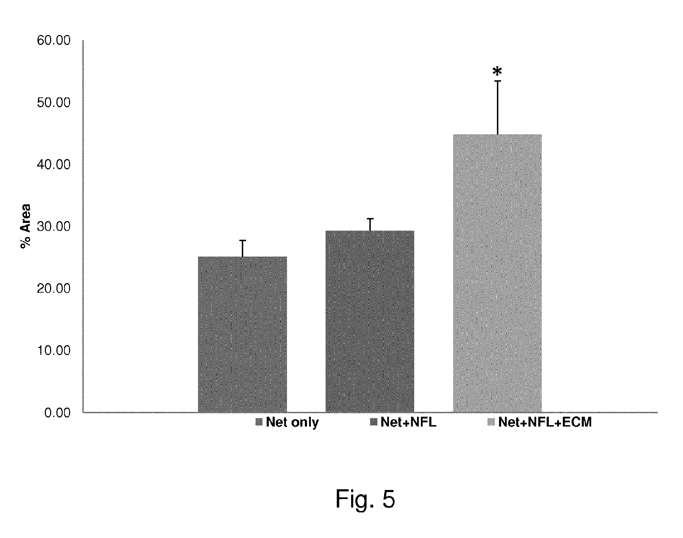

FIG. 5 is a histogram depicting the average percentages of area of the newly formed collagen around the implant in each type of devices. Two pathologists, blinded to the type of the implant, evaluated the percentage area of the newly formed collagen around the implant by using a computerized morphometric measurement. Note that rats which were implanted with a device containing ECM had significantly more widely spread collagen around the implant (45%) as compared with rats implanted with the mesh only (Net only; 25%, P<0.01) or the hybrid device which comprises the mesh + electrospun element (Net+NFL device; 29%, P<0.05). Also note that rats implanted with the hybrid device which comprises the mesh + electrospun element exhibit more collagen around the implant than rats implanted with the mesh alone.

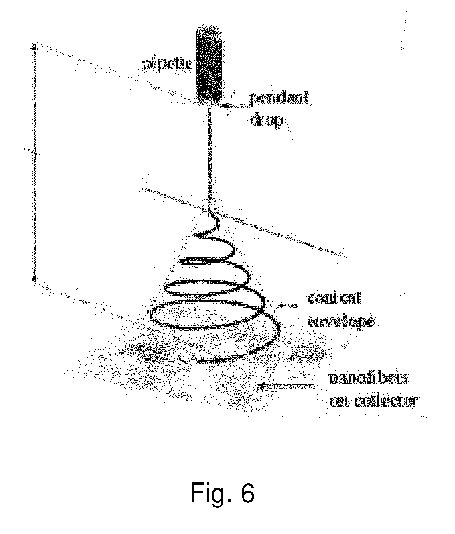

FIG. 6 is a schematic illustration depicting the process of electrospinning of a polymer from a pipette through a pendant drop, through a conical envelope onto a collector. In the electrospinning process used to generate the device of some embodiments of the invention, the nanofibers are placed on a mesh which is placed on the collector.

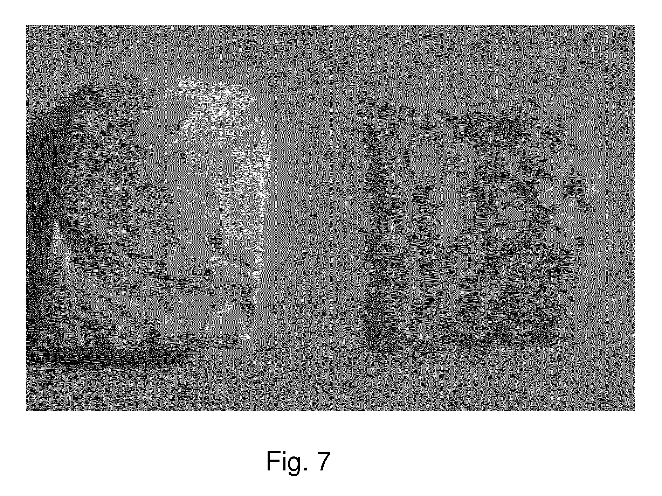

FIG. 7 depicts images of original (uncoated) mesh ("net" only; right side) and the mesh after being coated with an electrospun element on both sides ("net + NFL"; left side). The size of the mesh shown in 3x4 cm.

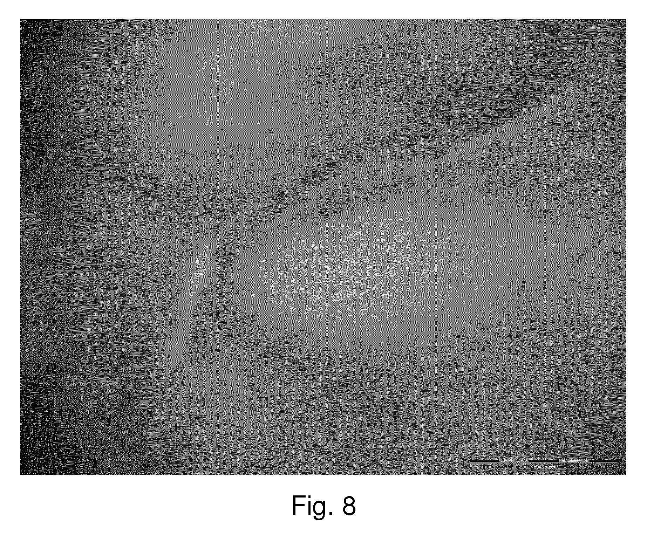

FIG. 8 is an image of showing high magnification of the mesh coated with the electrospun element shown in Figure 7 on the left side. Note the non-woven electrospun element covering the entire net (mesh).

### DESCRIPTION OF SPECIFIC EMBODIMENTS OF THE INVENTION

The present invention, in some embodiments thereof, relates to devices for surgical applications and to methods of generating same and using same for treating pathologies requiring reconstructive surgeries.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways.

The present inventors have uncovered a device suitable for use in various reconstructive surgeries. Thus, as shown in the Examples section which follows and in Figures 7 and 8, the present inventors have generated a hybrid device made of at least two layers, wherein one layer is of a mesh (also referred to as "net"), and a second layer is of an electrospun element (also referred to as a nanofiber layer "NFL"). The electrospun element was in contact with the mesh and filled all the holes (pores) of the mesh even in the deep layers of the mesh (Figures 7 and 8). Such device was found more suitable for reconstructive surgeries such as for repair of iatrogenic abdominal hernia than the mesh alone which is currently used in the clinic for reconstructive surgeries in terms of lower incidents of erosion of the implant and higher collagen areas around the implant (Figures 3A-D, Figures 4A-D and Figure 5). Moreover, as shown in Example 1 and Figures 4A-F and 5, when the device further included extracellular matrix which was deposited on and in the mesh-electrospun device, a better response of the recipient animal was observed with significantly higher areas of new collagen around the implant (45%) as compared with animals implanted with the mesh only (25%, P<0.01) or as compared a hybrid device which comprises the mesh + electrospun element (29%, P<0.05). These results suggest the use of the novel hybrid device for various surgical and clinical uses, in the treatment of pathologies requiring reconstructive surgery and tissue regeneration.

According to an aspect of some embodiments of the invention there is provided a device comprising at least two layers, the at least two layers being at least partially overlapping and contacting one another, wherein a first layer of the at least two layers comprises a mesh, and wherein a second layer of the at least two layers comprises an electrospun element, and wherein the device is devoid of an extracellular matrix (ECM) generated by mesenchymal progenitor cells (MPCs) which are characterized by a reduced differentiation potential into an adipogenic lineage by at least about 50% as compared to adipogenic differentiation potential of mesenchymal stem cells from an adult adipose source under identical assay conditions, and by an increased osteogenic differentiation potential by at least about 20% as compared to the osteogenic differentiation potential of adipose-derived MSCs under identical assays conditions.

As used herein the phrase "mesenchymal progenitor cells (MPCs)" refers to cells which are not terminally differentiated but exhibit a reduced differentiation potential to mesenchymal cell lineages as compared to naturally occurring mesenchymal stem cells derived from an adult tissue.

As used herein the phrase "adult tissue" refers to any tissue obtained from a post-natal subject, e.g., a post-natal human subject.

As used herein the phrase "differentiation potential" refers to the ability of an undifferentiated stem cell to differentiate into a certain cell lineage.

The differentiation potential can be evaluated by subjecting the stem cells to culture conditions (e.g., by culturing the stem cells in a culture medium supplemented with growth factors, minerals, cytokines and the like, in a flask with feeders cell, matrix, or in a suspension culture) which induce differentiation of the stem cells into a certain cell lineage, and comparing the degree (e.g., in percentages) of differentiation into the certain cell lineage between two populations of undifferentiated stem cells which are subjected to identical culture conditions. The percentage of differentiation can be calculated as the fraction of differentiated cells out of the initial number of undifferentiated cells (prior to their induction towards differentiation) in the culture, and such a fraction can be compared between two population of MSCs that are subjected to identical differentiation conditions.

Following in a non-limiting description of culture conditions suitable for inducing differentiation of undifferentiated mesenchymal stem cells into adipogenic cell lineage. MSCs [in BHK medium (composition of the medium is described below)] are seeded in tissue culture plates, for example, 2 x 10⁵ cells per well in a 6-well plate, or 5 x 10⁴ cells per well in a 24-well plate. On the following day, the cells are fed with "Basic medium" composed of (DMEM)/F-12 supplemented with 1% penicillin (10,000 U/mL)-streptomycin (10 mg/mL), 1 mM glutamine (all from biological industries, Beit Haemek, Israel) and 10 % lot specific FBS (Hyclone, Logan, UT, USA). For adipogenesis induction the "basic medium" is enriched with 10 µg/ml insulin, 0.5 mM 3-Isobutyl-1-methylxanthine (IBMX), 10⁻⁶ M dexamethasone and 100 x 10⁻⁶ M indomethacin (all from Sigma, Rehovot, Israel). The cells are cultured with this adipogenic medium for 28-30 days, without culture passaging, during which the medium is changed twice a week.

Quantization of the degree of differentiation into an adipogenic lineage can be performed by various methods (e.g., assays). For example, the number of fat drops per cells in the culture can be measured using for example, an image analysis system (e.g., ImagePro software). In addition, the Oil-Red O staining can be used to quantify the ability of mesenchymal stem cells or mesenchymal progenitor cells to differentiate into adipogenic lineage. Additionally or alternatively, the differentiation to adipogenic lineage can be determined by quantitative Real Time PCR using adipogenic differentiation markers as: Peroxisome- proliferator- activated receptor- y [PPAR γ; using e.g., primers specific to GenBank Accession Nos. NM_005037.5 (SEQ ID NO:1), NM_015869.4 (SEQ ID NO:2), NM_138711.3 (SEQ ID NO:3) and/or NM_138712.3 (SEQ ID NO:4)], CCAAT/enhancer-binding proteins beta [C/EBPß; using e.g., primers specific to GenBank Accession NO. NM_005194.2 (SEQ ID NO:5)], Leptin [using e.g., primers specific to GenBank Accession NO. NM_000230.2 (SEQ ID NO:6)] and adiponectin [using e.g., primers specific to GenBank Accession NO. M_001177800.1 (SEQ ID NO:7) and/or NM_004797.3 (SEQ ID NO:8)].

Following in a non-limiting description of culture conditions suitable for inducing differentiation of undifferentiated mesenchymal stem cells into osteogenic cell lineage. MSCs [in BHK medium (composition of the medium is described below)] are seeded in tissue culture plates, for example, 2 x 10⁵ cells per well are seeded in a 6-well plate, or 5 x 10⁴ cells per well are seeded in a 24-well plate in BHK medium which is used as the control medium throughout the experiment. On the following day, the cells are induced to differentiate into osteoblasts by enriching BHK medium with 10 mM β-glycerophosphate and 10⁻⁷ M dexamethasone (both from Sigma, Rehovot, Israel) (referred to as an "osteogenic medium", hereinafter). The cells are cultured with the osteogenic medium for 28-30 days, without culture passaging, during which the medium is changed twice a week.

The degree of osteogenic differentiation potential can be determined by amount of calcific deposition. Quantification of the calcific deposition can be determined by various methods, such as by Alizarin Red staining. Following 28 days of culturing in the osteogenic medium the cells are fixed and stained with Alizarin Red. Alizarin Red is used to identify calcium in tissue sections. Calcium forms an Alizarin Red S-calcium complex in a chelation process, and the end product is birefringent.

As described, the mesenchymal progenitor cells which produce the ECM that is excluded from the device of the claimed invention are characterized by a reduced potential to differentiate into adipogenic cell lineage as compared to the differentiation potential to the adipogenic lineage of mesenchymal stem cells from an adult adipose source under identical assay conditions and by increased potential to differentiate into osteoblast cell lineage as compared to the differentiation potential to the osteogenic lineage of mesenchymal stem cells from an adult adipose source under identical assay conditions.

As used herein the phrase "adult adipose source" refers to a lipoaspirate (e.g., raw lipoaspirate) obtained from human abdomen or thigh.

Following is a non-limiting description of isolation of mesenchymal stem cells from an adult adipose source. Raw human abdomen or thigh lipoaspirates are obtained, washed extensively with sterile phosphate-buffer saline (PBS) to remove contaminating debris. Washed aspirates are treated with 1% collagenase type I (Sigma, Rehovot, Israel) in PBS for 1 hour at 37°C with agitation. The collagenase is inactivated with an equal volume of DMEM/ 10% FBS/ 1 mM Glutamine/ 1% PenStrep/ 0.2 mg/ml Kanamycin and then is centrifuged for 10 minutes at 2000 rpm. The cellular pellet is resuspended in 160 mM Ammonium Chloride, incubated in room temperature for 10 minutes to remove red blood cells, neutralized with an equal volume of the BHK medium (see description of medium hereinabove) and filtered through a 100 mm mesh filter to remove large fat tissue debris. The filtrate is centrifuged as detailed above and plated onto conventional tissue culture flasks in BHK medium.

Mesenchymal stem cells from an adult adipose source are also commercially available. For example, Adipose-Derived Mesenchymal Stem Cells (derived form human Lipoaspirate) ATCC^{®} Number: PCS-500-011; Human Adipose-Derived Adult Stem Cells (catalog # ASC-F, ZEN-bio Inc., Research Triangle Park, NC 27709 U.S.A.); Human Mesenchymal Stem Cells from Adipose Tissue (hMSC-AT) (Catalogue numbers C-12977 and C-12978, PromoCell, GmbH, Sickingenstr. Heidelberg, Germany.

As described above, the device of some embodiments of the invention comprises at least two layers, which are at least partially overlapping and contacting one another.

According to some embodiments of the invention, the at least two layers are superposed and contacting one another.

As used herein the term "superposed" refers to layers which are either laid one on top of the other in a horizontal position, or layers which are adjacent or juxtaposed vertically.

According to some embodiments of the invention, the layer is a two-dimension layer.

According to some embodiments of the invention, the layer is a three-dimensional layer. According to some embodiments of the invention, the thickness of the layer is higher than the thickness of the fiber forming the layer. For example, the thickness of the layer can be at least one order of magnitude thicker than the thickness of the fiber forming the layer, e.g., at least 2 or 3 orders of magnitude.

According to some embodiments of the invention, each layer has sub-layers made of the same or different materials, wherein the sub layers can be horizontal layers or vertically stacked layers.

According to some embodiments of the invention, the device comprises at least 3 layers, at least 4 layers, at least 5 layers, at least 6 layers, at least 7 layers, at least 8 layers, at least 9 layers, at least 10 layers or more.

According to some embodiments of the invention, at least 2 of the layers are superposed and contacting one another.

According to some embodiments of the invention, the first layer and the second layer of the device contact each other by non-covalent bonds.

According to some embodiments of the invention, the first layer contacts the second layer by non-covalent bonds and the third layer contacts the second layer by non-covalent bonds.

According to some embodiments of the invention, the device comprises three layers, wherein the first layer is an electrospun element, the second layer is a mesh, and the third layer is an additional electrospun element (e.g., it can be a different or an identical electrospun element as the first layer), wherein the mesh is placed between the two electrospun layers.

According to some embodiments of the invention, when the device comprises three layers, wherein the mesh is in between the two electrospun layers, the first and third layers are connected to each other by non-covalent bonds which occur through the holes (pores) of the mesh.

According to some embodiments of the invention, the device comprises a single-coated mesh, wherein the coat comprises a layer of an electrospun element.

According to some embodiments of the invention, the device comprises a double-coated mesh, wherein each coat comprises a layer of an electrospun element.

According to some embodiments of the invention, the device comprises at least a double-coated mesh, e.g., a triple coated mesh or more, wherein each coat comprises a layer of an electrospun element.

The mesh the term "mesh" refers a composition constructed of a material having the appearance of a net (e.g., with holes, or pores).

According to some embodiments of the invention, the mesh is a synthetic mesh.

According to some embodiments of the invention, the mesh is suitable for medical or clinical applications, e.g., designed for surgical use, e.g., transplantation in a subject (e.g., a human or animal subject).

According to some embodiments of the invention, the mesh is of clinical or pharmaceutical grade.

According to some embodiments of the invention, the mesh is constructed from fibers having a diameter in the range of 1-100 mil (a thousandth of an inch), e.g., 5-50 mil, e.g., 5-40 mil, e.g., 5-30 mil, e.g., 5-20 mil; or in the range of 25-2540 µm (micrometer), e.g., about 100-1000 µm, e.g., about 125-500 µm.

According to some embodiments of the invention, the mesh is constructed by knitting of filaments, e.g., by simple knitting or by Warp knitting.

According to some embodiments of the invention, the mesh is made of a woven material.

According to some embodiments of the invention, the mesh is made of a nonwoven material.

The mesh can be produced from various materials such as polymers; polyester filament such as Dacron.TM. and Mersilene.TM. (Ethicon Inc., Somerville, N.J.); polyglycolic acid such as Dexon.TM. mesh; poly-4 hydroxybutyrate, Monofilament Polypropylene; polypropylene mesh such as Prolene.TM. (Ethicon Inc., Somerville, N.J.) and Marlex.TM. (C. R. Bard Inc.); silicone; polyethylene; polyamide; titanium; stainless steel; polymethylmethacrylate; nylon; silk; cotton; polyglactic acid such as Vicryl.TM. mesh (Ethicon Inc., Somerville, N.J.), poliglecaprone, polydioxone and expanded polytetrafluoroethylene such as DualMesh.TM., Mycromesh.TM., or other expanded PTFE (W. L. Gore and Associates); PDS.RTM., Vicryl.RTM., or Monocryl.RTM. and metal.

In some embodiments, the mesh may be multifilament polyester strands or monofilament polyester strands.

According to some embodiments of the invention, the mesh is made of a metal or a polymer.

The mesh may be characterized by various tensile strengths depending on the intended use. Non-limiting examples of tensile strengths of the mesh used by the device of some embodiments of the invention include a tensile strength in the range of about 100 N/mm² to about 4000 N/mm², e.g., about 450 N/mm² to about 2100 N/mm² ("N" = Newton).

According to some embodiments of the invention, the mesh is a supportive mesh.

As used herein the phrase "supporting mesh" refers to a mesh which may provide physical and/or mechanical support for a biological tissue when used for transplantation in a subject.

The supportive mesh of some embodiments of the invention may generally serve to provide strength and structural integrity to the biological tissue during its use in medical applications, thus serving as a reinforcement material. The reinforcement material may typically support the biological tissue and the surrounding tissue in general during wound repair and tissue closure.

For example, the supportive mesh can be the Prolift™ net available from Ethicon, Sommerville, NJ, USA; Prolift +M™, available from Ethicon, Sommerville, NJ, USA (Johnson and Johnson (J&J); AMS IntePro® Lite™ mesh (American Medical Systems, Inc.); Elevate AMS IntePro® Lite™ mesh (American Medical Systems, Inc.); Dynamesh® products (available from NBC Meshtech Inc); DEXON™ Mesh (Syneture); Safil® Mesh (B. BRAUN Medical Inc. Bethlehem, □PA □USA); SURUMESH® polypropylene mesh (SURU International Pvt. Ltd. C-6, Sona Udyog,

Andheri (E), Mumbai 400 069, India).

The mesh which is used by the device of some embodiments of the invention can be biodegradable, or non-biodegradable, or may have certain degree of biodegradability (e.g., partially biodegradable).

The biodegradability of a material can be determined by the degree of stability or degradability (e.g., being de-composed) in the physiological environment of a subject (e.g., within a human body, e.g., within tissues or body fluids of the subject). Commercially available meshes are characterized by the degree of biodegradability upon time following transplantation in a subject, and the tensile strength retention of the meshes are known by the manufacturer's of the commercially available meshes. For example, the tensile strength retention of the Safil® Mesh (B. BRAUN Medical Inc. Bethlehem, □PA □USA) is 50 % at 18 days post implantation; and the tensile strength retention of the Monomax® monofilament suture (B. BRAUN Medical Inc. Bethlehem, □PA □USA) is 50 % tensile strength after 90 days post implantation in a subject, retention of 0 % tensile strength after approx. 180 days, and complete mass absorption in around 13 months.

The degree of biodegradability or stability of the mesh depends on the intended used of the device, and the predicted rate of tissue regeneration, wound repair and/or tissue closure. Thus, for some applications, the mesh should remain stable for only short periods, such as up to 10-30 days from the day of transplantation within the subject, and for other applications, longer stability is needed for at least 30-60 days, or more.

According to some embodiments of the invention, the mesh maintains at least about 50%, e.g., about 55%, e.g., about 60%, e.g., about 65%, e.g., about 70%, e.g., about 75%, e.g., about 80%, e.g., about 85%, e.g., about 90%, e.g., about 95% of its structure, function (e.g., tensile strength) and dimension over a period of at least about one about week, at least one about one month, at least one about two months, at least one about three months, at least one about four months, at least one about five months, at least one about six months, at least one about seven months, at least about one year, or more.

According to some embodiments of the invention, the mesh is a non-biodegradable mesh.

As used herein the phrase "non-biodegradable material" refers to a substance (e.g., metal, polymer) which is essentially stable *i.e.,* non-degradable in the physiological environment of a subject (e.g., within a human body, e.g., within tissues or body fluids of the subject).

According to a specific embodiment, the non-biodegradable mesh maintains at least about 90 %, e.g., about 91%, about 92%, about 93%, about 94%, about 95%, about 96%, about 97%, about 98%, about 99%, 100% of its structure, function and dimension over a period of several years (e.g., at least 1-2 years) or over a life time in the body or tissue.

According to some embodiments of the invention, the non-biodegradable material is non-absorbable by the subject's body.

According to some embodiments of the invention, the mesh is a biodegradable mesh, which is being degraded within the tissue or body of the subject.

According to some embodiments of the invention, about 10%, 20%, 30%, 40%, 50%, 60%, 70%, 80%, 90% or 100% of the mesh is degraded following at least 60 days, e.g., following about 60-90 days, following about 60-120 days, following about 90-120 days, following about 90 days to six months, following about 3-4 months, following about 3-9 months, following about 3-12 moths.

According to some embodiments of the invention, the supportive mesh is biocompatible.

Selection of the supportive mesh may take into consideration the pore size, strength, permeability and flexibility of the material, as well as the structure and function of the surrounding tissue. For example, for use in applications involving load-bearing tissue, the supportive mesh may provide the appropriate tensile strength and flexibility to support the biological material and surrounding tissue during the formation of new tissue sufficient to support surrounding tissue. One of ordinary skill in the art can recognize the desired characteristics of the supportive mesh in selecting the optimal material.

Supportive meshes can be purchased from any commercial source and manipulated into the desired shape or form using techniques known in the art. For example, in forming the shape of a mesh, the supportive mesh can be an over- and under-weave that is heat tacked at each junction point.

In some embodiments of the invention, the supportive mesh may undergo a crosslinking treatment to alter the mechanical properties of the material. For example, the supportive mesh may undergo crosslinking treatment to increase the strength of the material for medical applications in load-bearing tissue.

The supportive mesh may be of any shape or size according to its application as a support to the biological material in medical applications. Selection of the appropriate shape or size of the supportive mesh is routine for one of ordinary skill in the art. For example, the supportive mesh may be in the form of fibers organized as a mesh or lattice. In some embodiments, the mesh may be comprised of a web, wherein the web is defined by a plurality of spaced apertures. The mesh or lattice can have various designs such as polygons (triangles, rectangles, etc.), circles, ovals, spirals, or any combination thereof. The spaces between the fibers of the mesh can vary according to the size of the mesh and the medical application (e.g., for implantation in a load-bearing tissue).

According to some embodiments of the invention, the pore size of the mesh is in the millimeter (mm) range, e.g., 1-100 mm, 1-90 mm, 1-80 mm, 1-70 mm, 1-60 mm, 1-50 mm, 1-40 mm, 1-30 mm, 1-20 mm, 1-10 mm, 1-2 mm, 3-4 mm and the like.

According to some embodiments of the invention, the pore size of the mesh is in the micrometer (µm) range, e.g., 100-5000 µm, e.g., 500-5000 µm, e.g., 500-4000 µm, e.g., 500-3000 µm, e.g., 500-2500 µm, e.g., 1000-2000 µm and the like.

According to some embodiments of the invention, the supportive mesh may be treated with an anti-infective agent. Non-limiting examples of anti-infective agents include, but are not limited to, anti-inflammatory agents, analgesic agents, local anesthetic agents, antispasmodic agents, or combinations thereof.

The addition of suitable anti-infective compounds to the surface of the mesh on the strands and junction points attack may inhibit the growth and proliferation of bacteria on and/or near the implant.

According to some embodiments of the invention, the supportive mesh may be treated with a protease inhibitor in order to alter its degradation rate. Non-limiting examples of protease inhibitors which can be used along with the invention include, but are not limited to, Aminoethylbenzenesulfonyl fluoride HCL, Aprotinin, Protease Inhibitor E-64, Leupeptin, Hemisulfate, EDTA, Disodium (0.025-0.10 um) or trypsin-like proteases, Pepstatin A (Aspartic Proteases), Mannistat (MMP2), or any combination thereof.

The above described treatments may be applied by methods known in the art, including, but not limited to, bathing, injecting, transfecting, bonding, coating, adding genetically modified cells and/or genetic material itself, or laminating.

Manufacturing of electrospun elements can be done by an electrospinning process which is well known in the art. Following is a non-limiting description of an electrospinning process. One or more liquefied polymers (*i.e.,* a polymer in a liquid form such as a melted or dissolved polymer) are dispensed from a dispenser within an electrostatic field in a direction of a rotating collector. The dispenser can be, for example, a syringe with a metal needle or a bath provided with one or more capillary apertures from which the liquefied polymer(s) can be extruded, e.g., under the action of hydrostatic pressure, mechanical pressure, air pressure and high voltage.

The rotating collector (e.g., a drum) serves for collecting the electrospun element thereupon. Typically, but not obligatorily, the collector has a cylindrical shape. The dispenser (e.g., a syringe with metallic needle) is typically connected to a source of high voltage, preferably of positive polarity, while the collector is grounded, thus forming an electrostatic field between the dispenser and the collector. Alternatively, the dispenser can be grounded while the collector is connected to a source of high voltage, preferably with negative polarity. As will be appreciated by one ordinarily skilled in the art, any of the above configurations establishes motion of positively charged jet from the dispenser to the collector. Inverse electrostatic configurations for establishing motions of negatively charged jet from the dispenser to the collector are also contemplated.

At a critical voltage, the charge repulsion begins to overcome the surface tension of the liquid drop. The charged jets depart from the dispenser and travel within the electrostatic field towards the collector. Moving with high velocity in the interelectrode space, the jet stretches and solvent therein evaporates, thus forming fibers which are collected on the collector, thus forming the electrospun element.

As used herein, the phrase "electrospun element" refers to an element of any shape including, without limitation, a planar shape and a tubular shape, made of one or more non-woven polymer fiber(s), produced by a process of electrospinning. When the electrospun element is made of a single fiber, the fiber is folded thereupon, hence can be viewed as a plurality of connected fibers. It is to be understood that a more detailed reference to a plurality of fibers is not intended to limit the scope of the present invention to such particular case. Thus, unless otherwise defined, any reference herein to a "plurality of fibers" applies also to a single fiber and vice versa. The electrospun element is also referred to as a nanofiber hereinafter.

The polymer fibers of the electrospun element can be arranged on a single layer, but, more preferably, the fibers define a plurality of layers hence form a three dimensional structure. The polymer fibers may have a general random orientation, or a preferred orientation, as desired e.g., when the fibers are collected on a cylindrical collector such as a drum, the polymer fibers can be aligned predominantly axially or predominantly circumferentially. Different layers of the electrospun element may have different orientation characteristics. For example, without limiting the scope of some embodiments of the invention to any specific ordering or number of layers, the fibers of a first layer may have a first predominant orientation, the fibers of a second layer may have a second predominant orientation, and the fibers of third layer may have general random orientation.

Various parameters involved in the electrospinning process may vary during the process in a continuous or non-continuous manner. These include, but not limited to: the velocity of the rotating collector, the characteristic of the electrostatic field vector (magnitude and/or direction), the size or shape of the capillary apertures of the dispenser (e.g., the size and/or cross-sectional shape of a needle attached to the dispenser), the dispensing flow rate of the at least one liquefied polymer the viscosity and/or concentration of the liquefied polymer and the concentration of charge control agent.

The characteristic of the electrostatic field vector can be varied during the electrospinning process in more than one way. In one preferred embodiment, the variation of the electric field is effected by varying, preferably continuously, the distance between the dispenser and the collector; in another preferred embodiment, the variation of the electric field is effected by varying, preferably continuously, the potential difference between the dispenser and the collector; in an additional embodiment, the variation of the electrostatic field is effected by varying both the distance and the potential difference in a substantially continues manner.

The polymer used in the electrospinning process for the manufacture of the electrospun element can be a natural, synthetic and/or biocompatible polymer.

The phrase "synthetic polymer" refers to polymers that are not found in nature, even if the polymers are made from naturally occurring biomaterials. Examples include, but are not limited to, aliphatic polyesters, poly(amino acids), copoly(ether-esters), polyalkylenes oxalates, polyamides, tyrosine derived polycarbonates, poly(iminocarbonates), polyorthoesters, polyoxaesters, polyamidoesters, polyoxaesters containing amine groups, poly(anhydrides), polyphosphazenes, and combinations thereof.

Suitable synthetic polymers for use in the present invention can also include biosynthetic polymers based on sequences found in collagen, elastin, thrombin, fibronectin, starches, poly(amino acid), poly(propylene fumarate), gelatin, alginate, pectin, fibrin, oxidized cellulose, chitin, chitosan, tropoelastin, hyaluronic acid, polyethylene, polyethylene terephthalate, poly(tetrafluoroethylene), polycarbonate, polypropylene and poly(vinyl alcohol), ribonucleic acids, deoxyribonucleic acids, polypeptides, proteins, polysaccharides, polynucleotides and combinations thereof.

The phrase "natural polymer" refers to polymers that are naturally occurring. Non-limiting examples of such polymers include, silk, collagen-based materials, chitosan, hyaluronic acid, alginate and albumin.

As used herein, the phrase "co-polymer" refers to a polymer of at least two chemically distinct monomers. Non-limiting examples of co-polymers include, PLA-PEG, PEGT/PBT, PLA-PGA PEG-PCL and PCL-PLA.

The phrase "biocompatible polymer" refers to any polymer (synthetic or natural) which when in contact with cells, tissues or body fluid of an organism does not induce adverse effects such as immunological reactions and/or rejections and the like. It will be appreciated that a biocompatible polymer can also be a biodegradable polymer.

According to some embodiments of the invention, the electrospun element is biodegradable.

The phrase "biodegradable polymer" refers to a synthetic or natural polymer which can be degraded (i.e., broken down) in the physiological environment such as by proteases. Biodegradability depends on the availability of degradation substrates (*i.e.,* biological materials or portion thereof which are part of the polymer), the presence of biodegrading materials (e.g., microorganisms, enzymes, proteins) and the availability of oxygen (for aerobic organisms, microorganisms or portions thereof), carbon dioxide (for anaerobic organisms, microorganisms or portions thereof) and/or other nutrients. Degradable polyesters are one of the widely used synthetic materials for electrospinning because they are biodegradable with metabolizable degradation products. The degradation rate of polyester can be controlled by changing the constitute of the polymer. Examples of biodegradable polymers include, but are not limited to, collagen, fibrin, hyaluronic acid, polylactic acid (PLA), polyglycolic acid (PGA), polycaprolactone (PCL), polydioxanone (PDO), trimethylene carbonate (TMC), calcium sulfate, polyethyleneglycol (PEG), Collagen, PEG-DMA, Alginate, Hydroxyapatite, chitosan, and/or copolymers thereof and/or mixtures thereof. For example, PLGA - Poly(lactic -co-glycolic) acid is the copolymer of both PGA and PLA, being the most popular synthetic polymer for tissue engineering applications because of its excellent biocompatibility and variable degradability obtained by controlled the ratio of PGA:PLA within the copolymer. PCL is a crystalline, biodegradable polymer, which is easily fabricated, and when electrospun exhibits good mechanical properties.

According to some embodiments of the invention, the electrospun element comprises polycaprolactone (PCL).

According to some embodiments of the invention, the electrospun element comprises polycaprolactone (PCL) and poly(lactic-co-glycolic acid) (PLGA), and/or combination thereof (e.g. PCL/PLGA 1:6).

According to some embodiments of the invention, the liquefied polymer can be made of one polymer or more, each can be a polymer or a co-polymer such as described hereinabove.

According to some embodiments of the invention, the liquefied polymer is a mixture of at least one biocompatible polymer and a co-polymer (either biodegradable or non-biodegradable).

As used herein the phrase "bioactivity" refers to the ability to stimulate host cell restoration and tissue remodeling. A good bioactivity can be evaluated by the ability to induce host tissue integration and ability of biodegradation or absorption when replaced by the host tissue.

According to some embodiments of the invention, the electrospun element comprises a nonwoven nanofiber.

According to some embodiments of the invention, the electrospun element comprises oriented fibers.

According to some embodiments of the invention, the electrospun element comprises non-oriented fibers.

According to some embodiments of the invention, the electrospun element comprises an active ingredient such as drug molecules, labels (e.g., a detectable moieties) attached thereto.

The device of some embodiments of the invention is homogenous, and uniform, with minimal batch to batch variations.

According to an aspect of some embodiments of the invention, there is provided a method of generating the device of some embodiments of the invention. The method comprising electrospinning a polymeric solution onto a mesh, thereby obtaining a layer of an electrospun element over a layer of the mesh, thereby generating the device.

According to some embodiments of the invention, the electrospun element adheres to the mesh by physical forces.

Following is a non-limiting description of generating a device which comprises the electrospun element and the mesh according to some embodiments of the invention.

The mesh (e.g., in the shape of a disc, but a mesh of any other shape can be used) are preferably treated with plasma with the aim of making the surface more compatible to the electrospun fibers and as a sterilization aid. For example, oxygen plasma can be used at 18 W for 10 minutes.

The treated mesh is then placed on the plate collector for the electrospinning process. Figure 6 schematically illustrates the electrospinning process. The electrospinning can be performed at room temperature (∼25 °C) and a relative humidity of 40-50%. In a non-limiting example, the spinning parameters can include: electrostatic field of approximately 0.7 kV/cm and a distance between the spinneret (metal pipette needle) and aluminum collector plate of 13 cm. The flow rate of the solutions can be 3 ml/hour (controlled by a syringe pump). The fibers are collected on a mesh that is placed on top of a slowly horizontal rotating plate collector (e.g., a 16 mm diameter mesh disc, e.g., of a Prolift net).

The fibers are electrospun on the upper side of the mesh (disc) to a thickness of about 5-100 µm, e.g., about 10-90 µm, e.g., about 5-20 µm, e.g., about 5-40 µm, e.g., about 20-80 µm, e.g., about 40-70 µm, e.g., about 40-60 µm, e.g., about 20-70 µm, e.g., about 20-50 µm, e.g., about 30 µm, e.g., about 50 µm. Then the mesh, which includes the electrospun element, can be removed from the collector, e.g., using a scalpel. Such a hybrid device comprises a layer of mesh and a layer of an electrospun element (fibers). It is noted that there is some adhesion of the fibers to the mesh (net) so that they stay together. However, caution should be used to avoid separation of the layers.

In order to increase the adhesion between the mesh and the electrospun element, the mesh with the electrospun element attached thereto can be placed again on the collector, and the electrospinning is repeated on the other side of the mesh (disc), resulting in a mesh covered in both sides with electrospun elements, e.g., a double-coated mesh. The layers of the electrospun element stick to each other by non-covalent bonding occurring between the electrospun elements, thereby producing a robust coating of fibers on the mesh.

Figure 7 depicts images of the netting before (right side) and after (left side) coating with fibers. Figure 8 depicts high magnification of the double-coated mesh shown in Figure 7 (left side) with the electrospun element on both sides of the mesh. It is noted that the electrospun element is a non-woven material which covers all holes (pores) of the entire mesh.

According to some embodiments of the invention, the device further comprises an extracellular matrix with the proviso that the ECM is not generated by mesenchymal progenitor cells, which are characterized by a reduced differentiation potential into an adipogenic lineage by at least about 50% as compared to differentiation potential of mesenchymal stem cells from an adult adipose source under identical assay conditions, and by an increased osteogenic differentiation potential by at least about 20% as compared to the osteogenic differentiation potential of adipose-derived MSCs under identical assays conditions.

It should be noted that ECM generated by MSCs which are characterized by at least 20% increased differentiation potential to osteogenic cell lineage as compared to the osteogenic differentiation potential of mesenchymal stem cells from an adult adipose source under identical assay conditions but which are not characterized by at least 50% reduced differentiation potential to the adipogenic cell lineage as compared to the adipogenic differentiation potential of mesenchymal stem cells from an adult adipose source under identical assay conditions (i.e., MSC with normal differentiation potential or up to about 5-50% reduced differentiation potential to the adipogenic cell lineage) are not to be excluded from the claimed device.

Thus, the MSC which produce the ECM which is used by the device and method of some embodiments of the invention may have normal differentiation potential to adipogenic or osteogenic lineages as compared to the differentiation potential of MSC from an adult adipose tissue (e.g., AD5T MSCs or any other commercial MSC from adult adipose tissue, e.g., ATCC^{®} Number: PCL-500-011), or may have up to about 50 % reduced differentiation potential to adipogenic lineage, e.g., up to about 45%, up to about 40%, up to about 35%, up to about 30%, up to about 25%, up to about 20%, up to about 15%, up to about 10%, up to about 5% reduced differentiation potential to adipogenic lineage as compared with the differentiation potential of MSC from an adult adipose tissue under identical assay conditions.

As used herein the phrase "mesenchymal stem cells (MSCs)" refers to cells derived from an adult tissue which are capable of differentiation into at least cells of an osteogenic lineage (e.g., osteoblasts), cells of an adipogenic lineage (e.g., adipose cells), and cells of a chondrogenic lineage (e.g., chondrocytes).

Mesenchymal stem cells give rise to one or more mesenchymal tissues (e.g., adipose, osseous, cartilaginous, elastic and fibrous connective tissues, myoblasts) as well as to tissues other than those originating in the embryonic mesoderm (e.g., neural cells) depending upon various influences from bioactive factors such as cytokines.

Mesenchymal stem cells (MSC) can be isolated from various sources including: embryonic yolk sac, placenta, umbilical cord, fetal and adolescent skin, peripheral blood, cord blood, bone marrow, human embryonic stem cells, induced pluripotent stem cells (iPS) and other tissues such as fat. The abundance of MSCs in the bone marrow (BM) far exceeds their abundance in other tissues and as such isolation from BM is often preferred.

For example, bone marrow derived MSCs may be obtained from iliac crest, femora, tibiae, spine, rib or other medullar spaces (Dominici, M et al., 2001. Bone marrow mesenchymal cells: biological properties and clinical applications. J. Biol. Regul. Homeost. Agents. 15: 28-37; which is incorporated herein by reference in its entirety).

Methods of isolating, purifying and expanding mesenchymal stem cells (MSCs) are known in the arts and include, for example, those disclosed by Caplan and Haynesworth in U.S. Pat. No. 5,486,359 and Jones E.A. et al., 2002, Isolation and characterization of bone marrow multipotential mesenchymal progenitor cells, Arthritis Rheum. 46(12): 3349-60.

For example, bone marrow derived MSCs can be obtained as follows. BM aspirates (usually 20 ml) are diluted with equal volumes of Hank's balanced salt solution (HBSS; GIBCO Laboratories, Grand Island, NY, USA) and layering the diluted cells over about 10 ml of a Ficoll column (Ficoll-Paque; Pharmacia, Piscataway, NJ, USA). Following 30 minutes of centrifugation at 2,500 x g, the mononuclear cell layer is removed from the interface and suspended in HBSS. Cells are then centrifuged at 1,500 x g for 15 minutes and resuspended in a complete medium (MEM, α medium without deoxyribonucleotides or ribonucleotides; GIBCO); 20 % fetal calf serum (FCS) derived from a lot selected for rapid growth of MSCs (Atlanta Biologicals, Norcross, GA); 100 units/ml penicillin (GIBCO), 100 µg/ml streptomycin (GIBCO); and 2 mM L-glutamine (GIBCO). Resuspended cells are plated in about 25 ml of medium in a 10 cm culture dish (Corning Glass Works, Corning, NY) and incubated at 37 °C with 5 % humidified CO₂. Following 24 hours in culture, nonadherent cells are discarded, and the adherent cells are thoroughly washed twice with phosphate buffered saline (PBS). The medium is replaced with a fresh complete medium every 3 or 4 days for about 14 days. Adherent cells are then harvested with 0.25 % trypsin and 1 mM EDTA (Trypsin/EDTA, GIBCO) for 5 min at 37 °C, replated in a 6-cm plate and cultured for another 14 days. Cells are then trypsinized and counted using a cell counting device such as for example, a hemocytometer (Hausser Scientific, Horsham, PA). Cultured cells are recovered by centrifugation and resuspended with 5 % DMSO and 30 % FCS at a concentration of 1 to 2 X 10⁶ cells per ml. Aliquots of about 1 ml each are slowly frozen and stored in liquid nitrogen.

To expand the mesenchymal stem cell fraction, frozen cells are thawed at 37 °C, diluted with a complete medium and recovered by centrifugation to remove the DMSO. Cells are resuspended in a complete medium and plated at a concentration of about 5,000 cells/cm². Following 24 hours in culture, nonadherent cells are removed and the adherent cells are harvested using Trypsin/EDTA, dissociated by passage through a narrowed Pasteur pipette, and preferably replated at a density of about 1.5 to about 3.0 cells/cm². Under these conditions, MSC cultures can grow for about 50 population doublings and be expanded for about 2000 fold [Colter DC., et al. Rapid expansion of recycling stem cells in cultures of plastic-adherent cells from human bone marrow. Proc Natl Acad Sci USA. 97: 3213-3218, 2000].

Mesenchymal stem cells (MSCs) are also available from various commercial sources. Non-limiting examples of commercially available MSCs include, human MSC derived from bone marrow cells [Catalogue Number: SCR108; Chemicon (Millipore)]; human MSC derived from human embryonic stem cells [Catalogue Number: SCC036 Chemicon (Millipore)]; and LT2 Immortalized Pancreatic Mesenchymal Cell Line [derived from isolated primary cultures of human fetal pancreatic fibroblasts (Catalogue Number: SCR013 Chemicon (Millipore)].

According to some embodiments of the invention, at least about 70%, at least about 75%, at least about 80%, at least about 85%, at least about 90%, at least about 95%, or more, e.g., 100% of the MSC cultures utilized by some embodiments of the invention exhibit negative staining for the hematopoietic stem cell markers CD34, CD11B, CD43 and CD45.

According to some embodiments of the invention, at least about 70% at least about 75%, at least about 80%, at least about 85%, at least about 90%, at least about 95%, or more, e.g., 100% of the MSC cultures utilized by some embodiments of the invention exhibit the CD105+/CD90+/CD73+/CD44+/CD29+ signature.

According to some embodiments of the invention, the ECM is deposited on the electrospun element and covers internal layers thereof (e.g., all layers of the electrospun element).

For the addition of ECM to the hybrid device, which comprises the mesh and the electrospun element, MSCs or other matrix-producing cells are seeded on the hybrid device and are further cultured thereon in a culture medium suitable for production of extracellular matrix (ECM). Prior to being seeded with the MSCs or other matrix producing cells, the hybrid device, which comprises the mesh and the electrospun element, is preferably sterilized using either Gas-treatment with ethylene oxide or plasma treatment for 1-2 seconds and then UV treatment for 6 -12 hour. The hybrid device, which comprises the mesh and the electrospun element, is preferably incubated for overnight at BHK medium to verify their sterilization.

Following is a non-limiting description of generating the hybrid device of some embodiments of the invention. MSCs or other matrix-producing cells are seeded on a hybrid device, which comprises the mesh and the electrospun element (e.g., 2 x 10⁵ MSC are seeded on the hybrid device having a diameter of 18 mm, and width of 30-100 µm). The concentration of MSC should be adjusted according to the desired size of the hybrid device. The hybrid device with the MSCs seeded thereon is cultured with in a suitable culture medium (e.g., BHK medium) for 2-4 weeks, during this period the medium is replaced twice a week. Following 3-4 weeks of MSC culturing on the hybrid device, the construct (which now comprises a mesh, electrospun element and extracellular matrix with cells producing same) is decellularized in order to eliminate all cells from the device, yet while leaving the ECM intact. Any known decellalurization or acellularization protocol may be used. For example, the hybrid device can be treated with Hypertonic solution (50 mM Tris-Hcl, 1 mM NaCl, 10 mM EDTA) for overnight with gentle agitation. The treatment is followed by PBS wash and then treatment by 1% Triton X-100 for 1-2 hours at room temperature, with gentle agitation, followed by 2 X PBS wash, and then treatment with 1 mg/ml DNase1 for 1 hour at 37°C.

According to an aspect of some embodiments of the invention, there is provided a method of treating a subject in need of a reconstructive surgery, comprising implanting the device of some embodiments of the invention in the subject in a manner suitable for reconstructing a tissue or an organ of the subject, thereby treating the subject in need of the reconstructive surgery.

The term "treating" refers to inhibiting, preventing or arresting the development of a pathology (disease, disorder or condition) and/or causing the reduction, remission, or regression of a pathology. Those of skill in the art will understand that various methodologies and assays can be used to assess the development of a pathology, and similarly, various methodologies and assays may be used to assess the reduction, remission or regression of a pathology.

As used herein, the term "subject" includes mammals, preferably human beings at any age which suffer from the pathology.

According to some embodiments of the invention, the subject suffers from a pathology selected from the group consisting of abdominal ventral hernia, inguinal hernia, pelvic floor defect (PFD), pelvic organ prolapse, and stress urinary incontinence, trauma, breast cancer, congenital breast defect, congenital abdominal wall defect, and congenital diaphragmatic hernia.

According to some embodiments of the invention, the device is used to reconstruct a tissue, by providing a mechanical strength while avoiding rejection by the recipient subject.

In cases of reconstruction surgeries, the repair of tissue can be evaluated by degree of compatibility of the graft by the host tissue, the generation of fibrous capsule around the foreign implant and the presence or absence of rejection of the implant.

The device of some embodiments of the invention can improve biological process of tissue regeneration or repair.

As used herein the phrase "improving a biological process of tissue regeneration or repair" refers to improving the rate, degree and/or quality of a biological process of tissue regeneration or repair of tissue regeneration by at least about 2 %, at least about 3 %, at least about 4 %, at least about 5 %, at least about 10 %, at least about 15 %, at least about 20 %, at least about 25 %, at least about 30 %, at least about 35 %, at least about 40 %, at least about 45 %, at least about 50 %, at least about 55 %, at least about 60 %, at least about 65 %, at least about 70 %, at least about 75 %, at least about 80 %, at least about 85 %, at least about 90 %, at least about 95 %, at least about 99 %, e.g., 100%, at least about 2 times, at least about 3-10 times, at least about 20, 30, 40, 50, 60-100, 200, 300, 400, 500-1000 times as compared to a tissue which is not being treated by the device of some embodiments of the invention, or as compared to a tissue treated under the same (e.g., identical) conditions by implanting a supportive mesh (e.g., a synthetic mesh) which is devoid of an electrospun element.

Methods of implanting grafts such as the device of some embodiments the invention into a subject are known in the art. For example, the device can be implanted subcutaneously, intradermally, into any body cavity (e.g., abdomen), into a wounded tissue, into an incision, and/or injected as a filler and as such can be used in many reconstructive surgeries in order to treat a subject in need of soft tissue regeneration or repair.

Following is a non-limiting list of uses of the device of some embodiments of the invention: surgical products such as vascular and arterial graft; valve and aorta replacement; lower urinary tract reconstruction; skin substitute or reconstruction; use as myocardial patch and heart valve substitute; venous graft reconstruction; various ortopedic applications as tendon and ligaments replacement, tendon and ligament reconstruction; use as a drug release device; use as a dermal filler(s), probably as a gel generated from the acellular device; use as a biosynthetic prosthesis for the repair of abdominal hernia, ventral abdominal hernia, inguinal hernia, diaphragmatic hernia and pelvic organ prolapse, vaginal prolapse repair (Vaginal Wall Prolapse), Pelvic Floor Repair, slings for the repair of stress urinary incontinence; abdominal wall reconstruction; breast reconstruction; and post-trauma reconstruction (e.g., extremities reconstruction).

According to some embodiments of the invention, the device is used as suburethral sling.

The most common treatment for stress urinary incontinence is the use of suburethral synthetic slings, which demonstrate a relatively high rate of extrusion of the repair material and infection at the surgery site. The device of some embodiments of the invention can reduce these side effects.

The device of some embodiments of the invention can be included in a kit/article of manufacture along with a packaging material and/or instructions for use in any of the above described methods, uses or applications.

The methods/uses described herein may be conducted batchwise.

As used herein the term "about" refers to ± 10 %.

The terms "comprises", "comprising", "includes", "including", "having" and their conjugates mean "including but not limited to".

The term "consisting of means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals there between.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., Eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorporated by reference as if fully set forth herein. Other general references are provided throughout this document. The procedures therein are believed to be well known in the art and are provided for the convenience of the reader. All the information contained therein is incorporated herein by reference.

### GENERAL MATERIALS AND EXPERIMENTAL METHODS

***Generation of a hybrid device which comprises a mesh and an electrospun element -*** Two types of fiber scaffolds [electrospun elements, also referred to as nanofibers layer (NFL) herein] were fabricated, one composed of polycaprolactone (PCL) and the other composed of 14.3% PCL and 85.7% poly(lactic-co-glycolic acid) (PLGA 85-15), (PCL/PLGA 1:6). Table 1 below describes the materials used to fabricate the scaffolds.

**Table 1, types of electrospun elements**

| ***Fiber Scaffold type*** | ***Polymer solution*** |
|---|---|
| PCL | 9 wt.% PCL 80K dissolved in a mixture of chloroform and dimethylformamide (DMF), 8:2 (w/w) |
| PLGA | 2.5wt.% PCL 80K + 15 wt.% PLGA 85-15 dissolved in a mixture of chloroform and DMF, 8:2 (w/w) |

Table 1.

**Table 2, types of hybrid devices**

| ***Hybrid devices (Scaffolds)*** |
|---|
| Net only (Prolift) |
| Net+ PCL |
| Net+ PCL/PLGA 1:6 |

Table 2.

### Electrospinning process

***Electrospinning set up and parameters:*** Fiber scaffolds were fabricated using a standard electrospinning process as illustrated in Figure 6. All experiments were conducted at room temperature (∼25 °C) and a relative humidity of 40-50%. The spinning parameters were as follow: the electrostatic field used was approximately 0.7 kV/cm and the distance between the spinneret (metal pipette needle) and aluminum collector plate was 13 cm. The flow rate of the solutions was 3 ml/hr (controlled by a syringe pump). The fibers were collected on 16 mm diameter Prolift net discs that were placed on top of a slowly horizontal rotating plate collector.

***Deposition process*/ *coating process -*** Discs of Prolift netting were first treated with plasma with the aim of making the surface more compatible to the fibers (and as sterilization aid). Oxygen plasma was used at 18 W for 10 minutes.

The treated discs were placed on the plate collector. Fibers were electrospun on the upper side of the discs to a thickness of about 30 µm. Then they were cut out with a scalpel. There is some adhesion of the fibers to the net so that they stay together, but it is a relatively weak adhesion and with handling may start to separate. Therefore, the discs were placed again on the collector and fibers were deposited on the other side of the discs. In this way some fibers from both sides of the net stick to each other producing a robust coating of fibers on the net. Figure 7 depicts images of the netting before and after coating with fibers. Figure 8 depicts high magnification of the mesh-electrospun element shown in Figure 7 (left size).

***Cells culture and ECM generation -*** Adipose derived AD5T (primary MSCs generated from thigh fat) cells were cultured in BHK medium, composed of Glasgow Minimum Essential Medium (GMEM) (GIBCO-Invitrogen, Paisley, UK) with glutamine and supplemented with 10% fetal bovine serum (FBS) (Hyclone, Logan, UT, USA), 1% penicillin (10,000 U/mL)-streptomycin (10 mg/mL) (Biological Industries, Beit Haemek, Israel), 1% non-essential amino acids (NEAA)x100, 1 mM sodium pyruvate, 0.75 mM 2-mercaptoethanol (all from GIBCO-Invitrogen) and 100 mg/ml L-ascorbic acid 2-phosphate (Sigma, Rehovot, Israel). Cells were routinely passaged every 3-4 days up to passages 12-15. For ECM generation, cells were cultured for 3-4 weeks with BHK medium, with no subsequent passaging and with medium replacement twice a week.

***Cells seeding -*** For cell seeding, the hybrid devices were sterilized, either by means of ethylene oxide gas-sterilization, or by UV sterilization for 6 -12 hours in a sterile hood. To validate their sterilization before seeding the cells, the devices were incubated for at least overnight in BHK medium and were examined for presence of contamination. Thereafter, 150,000 cells were seeded on the hybrid device [having dimensions of: 20 mm (millimeters) diameter; and 3-5 mm width] supplemented with BHK medium. Cells were cultured for 2-4 weeks and the medium replaced twice a week.

***Acellularization protocol -*** Following 2-4 weeks of cells culturing on the device, the hybrid construct was decellularized using the following method: the device was treated with hypertonic solution (50 mM Tris-Hcl, 1 mM NaCl, 10 mM EDTA) for overnight with gentle agitation. The treatment was followed by PBS wash and then treatment by 1% Triton X-100 for 1-2 hours at room temperature, with gentle agitation, followed by 2 X PBS wash, and then treatment with 1 mg/ml DNase1 for 1 hour at 37°C.

***Abdominal hernia model -*** Sprague-Dawley rats (300 grams each) were used. Animals were provided with food and water ad libitum. The light and dark cycle and room temperature were automatically controlled. The animals were housed under these conditions for 3-4 days before the experiments were conducted, so they would acclimatize. Animal care was in accordance with the guidelines of the Committee for the Supervision of Animal Experiments, Technion, Israel Institute of Technology.

After induction of general anesthesia (ketamine and xylazine), a midline incision was made on the abdomen. For creating a model for the repair of iatrogenic abdominal hernia, a 1 X 2 cm laparotomy defect was created, followed by suturing of the net or the hybrid device to the musculofascial abdominal wall edges using 5 Vicryl 4-0 (Ethicon) sutures. The inlay technique resulted in a musculofascial defect bridged by grafted device. The skin was closed with a continuous suture of Vicryl 4-0 (Ethicon). Rats were euthanized at 8 weeks after implantation, and the implants were carefully removed from adjacent tissues and examined by histological analysis.

***Histology -*** Tissue specimens were placed into formalin for fixation and then embedded in paraffin. Specimens were cut into serial sections, and representative sections underwent hematoxylin and eosin (H&E) and Masson's Trichrome (TC) stainings for examination of histomorphology and the detection of matrix collagens.

***Quantification of collagen formation -*** Two pathologists, blinded to the type of the implant, evaluated the percentage area of the newly formed collagen around the implant by using a computerized morphometric measurement (Mediacybernietic, MA, USA).

### EXAMPLE 1

### Experimental Results

Twenty rats were subjected to iatrogenic abdominal hernia, and implants were sutured to inlay the laparotomy defect in a way that the musculofascial defect was bridged by the grafted device. In the depicted experiments the implants included: (1) Net only - Prolift™ (Figures 1A and 1C; 7 rats, of which, 2 rats died and 5 rats were further examined); (2) Net+NFL device - Prolift™ coated with electrospun PCL:PLGA 1:6 (Figures 1B and 1D; 6 rats); and (3) Net+NFL+ECM device - Net+NFL with acellular AD5T derived ECM (Figures 1E and 1F; 7 rats). Figures 1A, 1B and 1E show the devices during implantation to the rats, and Figures 1C, 1D and 1F show the rats 8 weeks following implantation of the devices at euthanization.

Surgery was well tolerated in all animals. External recovery of the incision with no dehiscence of the abdominal scar could be seen in all the animals (Figures 2A and B). However, erosion of the implant through the abdominal scar was observed in some (∼50%-60%) of the rats implanted with Net (Figures 3A and 3C) or Net+NFL (Figures 3B and 3D, 40%), but not in the rats implanted with Net+NFL+ECM device (data not shown). In cases were erosion of the implant occurred, infection and crust was seen in the abdominal incision and implant was found to be superficially adhering to the abdominal incision. Examples for erosion are provided in Figures 3A-D.

At sacrifice, the implants were carefully removed from adjacent tissues and examined by histological analysis. Figures 4A-F demonstrate representative sections stained with Masson's Trichrome (TC) identifying matrix collagens. Two pathologists, blinded to the type of the implant, evaluated the percentage area of the newly formed collagen around the implant by using a computerized morphometric measurement. Comparison of the % area of collagen around the implant demonstrated that devices containing ECM had significantly more widely spread collagen around the implant (45%) as compared with rats implanted with Net only (25%, P<0.01) or Net+NFL device (29%, P<0.05) (Figure 5). These results indicate for the potential benefit of the Net+NFL and Net+NFL+ECM devices in reconstructive procedures.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention. To the extent that section headings are used, they should not be construed as necessarily limiting.

## Claims

1. A device comprising at least two layers, said at least two layers being at least partially overlapping and contacting one another, wherein a first layer of said at least two layers comprises a mesh, and wherein a second layer of said at least two layers comprises an electrospun element, and wherein the device is devoid of an extracellular matrix generated by mesenchymal progenitor cells, which are **characterized by** a reduced differentiation potential into an adipogenic lineage by at least about 50% as compared to differentiation potential of mesenchymal stem cells from an adult adipose source under identical assay conditions, and by an increased osteogenic differentiation potential by at least about 20% as compared to the osteogenic differentiation potential of adipose-derived MSCs under identical assays conditions.

2. The device of claim 1, further comprises extracellular matrix (ECM), with the proviso that said ECM is not generated by mesenchymal progenitor cells, which are **characterized by** a reduced differentiation potential into an adipogenic lineage by at least about 50% as compared to differentiation potential of mesenchymal stem cells from an adult adipose source under identical assay conditions, and by an increased osteogenic differentiation potential by at least about 20% as compared to the osteogenic differentiation potential of adipose-derived MSCs under identical assays conditions.

3. A method of generating the device of claim 1 or 2, comprising electrospinning a polymeric solution onto a mesh, thereby obtaining a layer of an electrospun element over a layer of the mesh, thereby generating the device.

4. The method of claim 3, wherein said electrospun element adheres to said mesh by physical forces.

5. The device of claim 1 or 2, or the method of claim 3 or 4, wherein said mesh is non-biodegradable.

6. The device of claim 1 or 2, or the method of claim 3 or 4, wherein said mesh is biodegradable.

7. The device of claim 1, 2, 5 or 6, or the method of claim 3, 4, 5, or 6, wherein said mesh comprises a biocompatible material.

8. The device of claim 1, 2, 5, 6 or 7, or the method of claim 3, 4, 5, 6 or 7, wherein said electrospun element comprises a biocompatible polymer.

9. The device of any of claims 1, 2 and 5-8, or the method of any of claims 3-8, wherein said electrospun element is biodegradable.

10. The device of any of claims 1, 2 and 5-9, or the method of any of claims 3-9, wherein said mesh is made of a nonwoven material.

11. The device of any of claims 1, 2 and 5-10, wherein said first layer and said second layer are connected to each other by non-covalent bonds.

12. The device of any of claims 1, 2 and 5-11, or the method of any of claims 3-11, wherein said electrospun element comprises an active ingredient attached thereto.

13. The device of any of claims 1, 2 and 5-12 for use in a method of treating a subject in need of a reconstructive surgery, the method comprising implanting the device in a subject in a manner suitable for reconstructing a tissue or an organ of the subject.

14. The device of claim 13, wherein said reconstructive surgery is for treating a pathology selected from the group consisting of abdominal ventral hernia, pelvic floor defect (PFD), pelvic organ prolapse, and stress urinary incontinence.

15. The device of any of claims 1, 2 and 5-12 for use as a suburethral sling.
